(19)

Europäisches Patentamt
European Patent Office
Office européen des brevets

(11) **EP 4 736 734 A1**

(12) **EUROPEAN PATENT APPLICATION**
published in accordance with Art. 153(4) EPC

(43) Date of publication:
06.05.2026 Bulletin 2026/19

(21) Application number: 24832017.8

(22) Date of filing: 26.06.2024

(51) International Patent Classification (IPC):
*A61B 1/00* (2006.01)       *A61B 1/07* (2006.01)

(52) Cooperative Patent Classification (CPC):
A61B 1/00; A61B 1/07

(86) International application number:
PCT/JP2024/023260

(87) International publication number:
WO 2025/005158 (02.01.2025 Gazette 2025/01)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 30.06.2023 JP 2023108686

(71) Applicant: Sony Olympus Medical Solutions Inc.
Hachioji-shi, Tokyo 192-0904 (JP)

(72) Inventors:
• IWANE, Tetsuaki
  Hachioji-shi, Tokyo 192-0904 (JP)
• DEGUCHI, Tatsuya
  Hachioji-shi, Tokyo 192-0904 (JP)
• YAMAMOTO, Takahiro
  Hachioji-shi, Tokyo 192-0904 (JP)

(74) Representative: D Young & Co LLP
3 Noble Street
London EC2V 7BQ (GB)

(54) **MEDICAL OBSERVATION SYSTEM, FILTER, AND OPTICAL VIEWING TUBE**

(57)     A medical observation system 1 includes: a light source device 3 configured to emit first light; an imaging element 513 configured to image second light, the second light being return light of the first light from an observation target OB to which the first light has been applied; and a filter 11 disposed on an observation optical path P0 including: a first optical path P1 through which the first light propagates from the light source device 3 to the observation target OB; and a second optical path P2 through which the second light propagates from the observation target OB to the imaging element 513, and configured to partially, substantially, or completely inhibit unnecessary light containing auto-fluorescent light generated from a member forming the observation optical path P0 by at least one of the first light and the second light.

FIG.1

## Description

Field

The present disclosure relates to a medical observation system, a filter, and an optical visual tube. Background

A medical observation system has been known in which first light is applied to an observation target (subject such as human) and fluorescent light emitted from a substance contained in the observation target (hereinafter, referred to as observation target fluorescent light) by applying the excitation light is observed (e.g., see Patent Literature 1). The first light contains excitation light, which is narrowband light emitted from a light source device, and visible light such as white light, which is broadband light.

According to such fluorescent light observation, a tissue state difficult to be recognized with white light can be grasped via observation target fluorescent light. Therefore, fluorescent light observation can be used for various purposes and applications such as identification of a lesion portion.

Citation List

Patent Literature

Patent Literature 1: JP 2021-132695 A

Summary

Technical Problem

Incidentally, a medical observation system includes an observation optical path including a first optical path and a second optical path. First light propagates through the first optical path from a light source device to an observation target. Second light propagates through the second optical path. The second light is return light (including observation target fluorescent light) of the first light from the observation target to which the first light has been applied. Then, auto-fluorescent light is generated from a member forming the observation optical path by applying the first and second beams of light to the member at the time when the first and second beams of light propagate through the observation optical path. Such auto-fluorescent light has a wavelength band including the wavelength band of the observation target fluorescent light, which is a target of fluorescent light observation, and serves as noise in observing fluorescent light.

Thus, there is a demand for a technique that enables good fluorescent light observation.

The present disclosure has been made in view of the above, and an object thereof is to provide a medical observation system, a filter, and an optical visual tube which enable good fluorescent light observation Solution to Problem

To solve the above-described problem and achieve the object, a medical observation system according to the present disclosure includes: a light source device configured to emit first light; an imaging element configured to image second light, the second light being return light of the first light from an observation target to which the first light has been applied; and a filter disposed on an observation optical path including: a first optical path through which the first light propagates from the light source device to the observation target; and a second optical path through which the second light propagates from the observation target to the imaging element, and configured to partially, substantially, or completely inhibit unnecessary light containing auto-fluorescent light generated from a member forming the observation optical path by at least one of the first light and the second light.

Moreover, a filter according to the present disclosure is disposed on an observation optical path, the observation optical path including: a first optical path through which first light propagates from a light source device configured to emit the first light to an observation target; and a second optical path through which second light propagates, the second light being return light of the first light from the observation target to which the first light has been applied, and configured to partially, substantially, or completely inhibit unnecessary light containing auto-fluorescent light generated from a member forming the observation optical path by at least one of the first light and the second light.

Moreover, an optical visual tube according to the present disclosure applies first light to an observation target and capturing second light, the second light being return light of the first light from the observation target to which the first light has been applied, and includes: a part of a first optical path through which the first light propagates from a light source device configured to emit the first light to the observation target; a part of a second optical path through which the second light propagates; and a filter configured to partially, substantially, or completely inhibit unnecessary light containing auto-fluorescent light generated from a member forming an observation optical path including the first optical path and the second optical path by at least one of the first light and the second light.

Advantageous Effects of Invention

The medical observation system, the filter, and the optical visual tube according to the present disclosure enables good fluorescent light observation.

Brief Description of Drawings

FIG. 1 illustrates a configuration of a medical observation system according to a first embodiment.
FIG. 2 illustrates the shape of a filter.
FIG. 3 illustrates the function of the filter.
FIG. 4 illustrates the function of the filter.
FIG. 5 illustrates the function of the filter.
FIG. 6 illustrates a method of calculating an S/N ratio.
FIG. 7 illustrates effects of the first embodiment.
FIG. 8 illustrates a configuration of a medical observation system according to a second embodiment.
FIG. 9 illustrates the function of a second filter in a case of short-wave excitation.
FIG. 10 illustrates the function of the second filter in the case of short-wave excitation.
FIG. 11 illustrates the function of the second filter in the case of short-wave excitation.
FIG. 12 illustrates the function of the second filter in a case of long-wave excitation.
FIG. 13 illustrates the function of the second filter in the case of long-wave excitation.
FIG. 14 illustrates the function of the second filter in the case of long-wave excitation.
FIG. 15 illustrates Variation 1 of the first and second embodiments.
FIG. 16 illustrates Variation 1 of the first and second embodiments.
FIG. 17 illustrates Variation 1 of the first and second embodiments.
FIG. 18 illustrates Variation 2 of the first and second embodiments.
FIG. 19 illustrates Variation 3 of the first and second embodiments.
FIG. 20 illustrates Variation 4 of the first and second embodiments.
FIG. 21 illustrates Variation 4 of the first and second embodiments.
FIG. 22 illustrates Variation 5 of the first and second embodiments.
FIG. 23 illustrates Variation 5 of the first and second embodiments.
FIG. 24 illustrates Variation 6 of the first and second embodiments.
FIG. 25 illustrates Variation 7 of the first and second embodiments.
FIG. 26 illustrates Variation 8 of the first and second embodiments.
FIG. 27 illustrates Variation 8 of the first and second embodiments.

Description of Embodiments

Embodiments for carrying out the present disclosure (hereinafter, embodiments) will be described below with reference to the drawings. Note that the present disclosure is not limited by the embodiments to be described below. Moreover, in the drawings, the same reference signs are attached to the same parts.

(First Embodiment)

[Configuration of Medical Observation System]

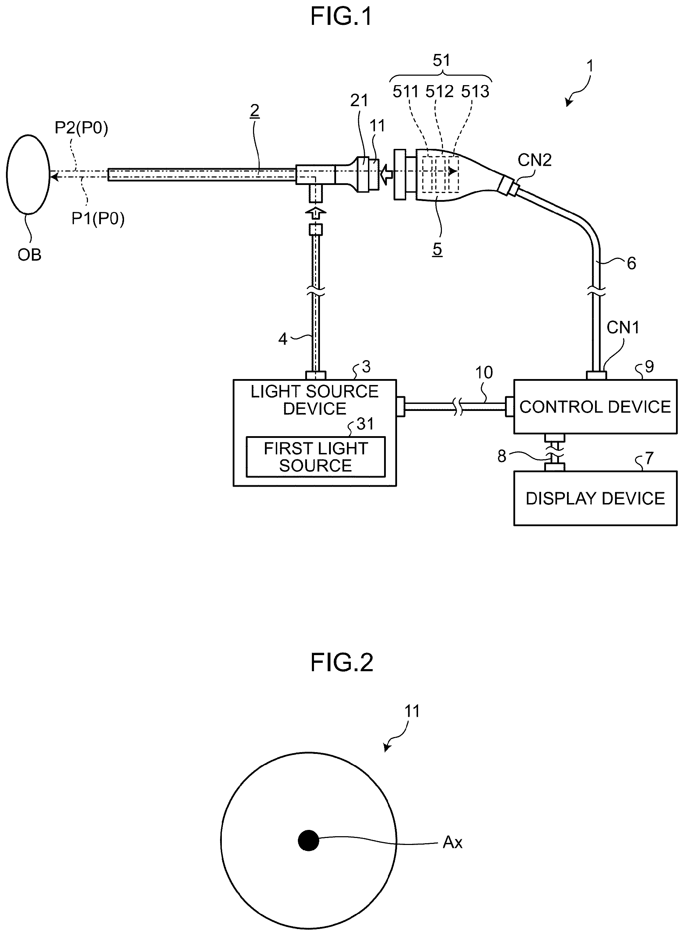

FIG. 1 illustrates a configuration of a medical observation system 1 according to a first embodiment.
In the first embodiment, the medical observation system 1 is a medical endoscope system for observing an observation target OB (inside of living body) with an endoscope. As illustrated in FIG. 1, the medical observation system 1 includes an insertion portion 2, a light source device 3, a light guide 4, a camera head 5, a first transmission cable 6, a display device 7, a second transmission cable 8, a control device 9, a third transmission cable 10, and a filter 11.
The insertion portion 2 corresponds to an optical visual tube according to the present disclosure. In the first embodiment, the insertion portion 2 includes a rigid endoscope. That is, the insertion portion 2 has an elongated shape. The insertion portion 2 is entirely rigid, or has a flexible part and the other rigid part. The insertion portion 2 is to be inserted into the observation target OB. An optical system is provided in the insertion portion 2. The optical system includes one or a plurality of lenses, and collects second light (subject image) to be described later from the observation target OB.
One end of the light guide 4 is connected to the light source device 3. Then, the light source device 3 includes a first light source 31 (FIG. 1) that supplies first light to the end of the light guide 4 under the control of the control device 9. In the embodiment, excitation light that excites a substance contained in the observation target OB is adopted as the first light. Note that the first light source 31 may include a light emitting diode (LED) or a semiconductor laser. Furthermore, one or a plurality of first light sources 31 that emit the first light may be provided.

Here, examples of the substance contained in the observation target OB to be excited by the excitation light can include medical agents and fluorescent dyes imparted to the observation target OB and fluorescent substances derived from the observation target OB constituting the observation target OB itself.

Examples of the above-described medical agents to be imparted to the observation target OB can include "5-ALA (PP-IX)", "ADS780WS", "ADS830WS", "aggregation-induced emission dots allophycocyanin (APC)", "boron-dipyrromethane (BODIPY)", "CLR 1502", "Flavins", "fluorescamine", "Fluorescein", "fluoro-gold", "green fluorescence protein", "indocyanine green (ICG)", "IRDye 78", "IR-PEG nanoparticles", "Isothiocyanate", "rose Bengal", "SGM-101", and "trypan blue".

Furthermore, examples of the above-described fluorescent dyes to be imparted to the observation target OB can include "coumarine", "Cy3", "DyLight 547", "GE3126", "metal nanoclusters", "oxacarbocyanine", "Rhodamine", "Riboflavin", "fluorescein", "Alexa Fluor 660", "Alexa Fluor 680", "Alexa Fluor 700", "Cy5", "Cy5.5", "Dy677", "Dy682", "Dy752", "DyLight 647", "HiLyte Fluor 647", "HiLyte Fluor 680", "IRDye 700DX", "methylene blue", "Porphyrins", "Porphysomes", "VivoTag-680", "VivoTag-S680", "Alexa Fluor 750", "Alexa Fluor 790", "carbocyanine", "conjugated copolymers", "CW800-CA", "Cy7", "Cy7.5", "cyanine dyes", "Dy780", "HiLyte Fluor 750", "Indocarbocyanine", "IR-786", "IRDye 800CW", "IRDye 800RS", "IRDye 800BK", "Nervelight", "OTL-38 (Pafolacianine)", "Polymethine", "VivoTag-S750", "Xanthene", and "LUM-015".

Moreover, examples of the fluorescent substances derived from the observation target OB constituting the observation target OB itself can include "collagen", "elastin", and "NADH".

Note that, although the light source device 3 is separated from the control device 9 in the first embodiment, this is not a limitation. A configuration provided in the same casing as the control device 9 may be adopted.

The end of the light guide 4 is detachably connected to the light source device 3. Furthermore, the other end of the light guide 4 is detachably connected to the insertion portion 2. Then, the light guide 4 propagates the first light supplied from the light source device 3 (first light source 31) from one end to the other end, and supplies the first light to the insertion portion 2. The first light supplied to the insertion portion 2 is emitted from a distal end of the insertion portion 2, and applied to the observation target OB. The optical system in the insertion portion 2 collects the second light (subject image), which is return light of the first light from the observation target OB. The second light has been applied to the observation target OB. In addition to excitation light reflected by the observation target OB, the second light includes fluorescent light emitted from a substance contained in the observation target OB (hereinafter, referred to as observation target fluorescent light) at the time when the excitation light is applied to the observation target OB and the substance is excited.

The camera head 5 is detachably connected to a proximal end (eyepiece portion 21 (FIG. 1)) of the insertion portion 2 via the filter 11. As illustrated in FIG. 1, the camera head 5 includes an imaging unit 51.

As illustrated in FIG. 1, the imaging unit 51 includes an excitation light cut filter 511, a lens unit 512, and an imaging element 513.

The excitation light cut filter 511 partially, substantially, or completely inhibits excitation light contained in the second light (subject image) collected by the insertion portion 2.

Note that the excitation light cut filter 511 may be provided not in the camera head 5 but in the insertion portion 2 (e.g., eyepiece portion 21).

The lens unit 512 forms an image of the second light (subject image) on a light receiving surface (imaging surface) of the imaging element 513. The second light (subject image) has been collected by the insertion portion 2 to pass through the excitation light cut filter 511.

The imaging element 513 includes a charge coupled device (CCD) or a complementary metal oxide semiconductor (CMOS), which receive the second light (subject image) and convert the second light (subject image) into an electric signal. The lens unit 512 has formed an image of the second light (subject image) after the second light passed through the excitation light cut filter 511.

Then, the imaging unit 51 outputs a pixel signal obtained by the imaging under the control of the control device 9.

One end of the first transmission cable 6 is detachably connected to the control device 9. Furthermore, the other end of the first transmission cable 6 is detachably connected to the camera head 5. Then, the first transmission cable 6 transmits the pixel signal output from the imaging unit 51 to the control device 9, and transmits each of, for example, a control signal, a synchronization signal, a clock, and power, which have been transmitted from the control device 9, to the camera head 5.

Note that, for example, a pixel signal may be transmitted from the camera head 5 to the control device 9 via the first transmission cable 6 by an optical signal or an electric signal. A control signal, a synchronization signal, and a clock may also be transmitted similarly from the control device 9 to the camera head 5 via the first transmission cable 6.

The display device 7 includes a display formed of, for example, liquid crystal or organic electro luminescence (EL). The display device 7 displays an image based on a video signal from the control device 9 under the control of the control device 9.

One end of the second transmission cable 8 is detachably connected to the display device 7. Furthermore, the other end of the second transmission cable 8 is detachably connected to the control device 9. Then, the second transmission cable 8 transmits the video signal that has been processed by the control device 9 to the display device 7.

The control device 9 includes a controller such as a central processing unit (CPU) and a micro processing unit (MPU), and integrally controls operations of the light source device 3, the camera head 5, and the display device 7. Note that the control device 9 may include not only the CPU and the MPU but an integrated circuit such as an application specific integrated circuit (ASIC), a fieldprogrammable gate array (FPGA), and a graphics processing unit (GPU).

Specifically, the control device 9 generates a video signal by performing various pieces of image processing on a pixel signal acquired from the camera head 5 via the first transmission cable 6, and outputs the video signal to the display device 7 via the second transmission cable 8. Then, the display device 7 displays an image based on the video signal. Furthermore, the control device 9 outputs, for example, a control signal to the camera head 5 and the light source device 3 via the first and third transmission cables 6 and 10.

One end of the third transmission cable 10 is detachably connected to the light source device 3. Furthermore, the other end of the third transmission cable 10 is detachably connected to the control device 9. Then, the third transmission cable 10 transmits a control signal from the control device 9 to the light source device 3.

FIG. 2 illustrates the shape of the filter 11. Specifically, FIG. 2 illustrates the filter 11 viewed along an optical axis Ax of light incident on the filter 11.

The filter 11 has a circular shape in plan view (FIG. 2), and is disposed between the eyepiece portion 21 and the camera head 5 such that the optical axis Ax is located at the center of the circular shape (FIG. 1). Specifically, the filter 11 is detachably connected to an eyepiece portion 21. Note that the filter 11 may have not only the circular plan shape but another plan shape. For example, the filter 11 may have a rectangular plan shape with an inscribed circle having the optical axis Ax as the center of the circle.

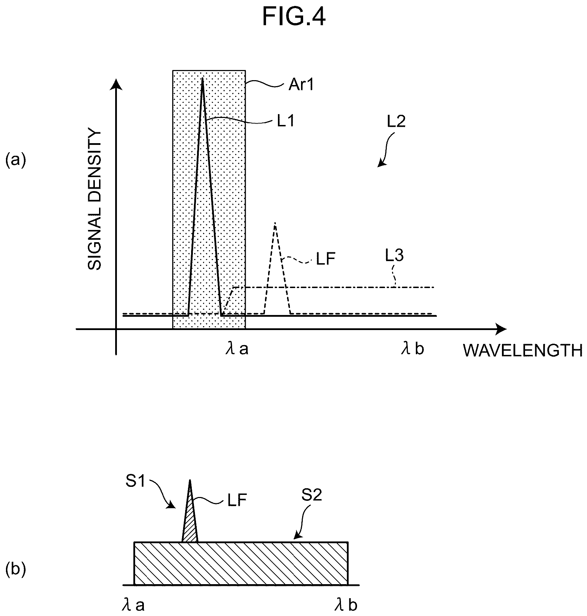

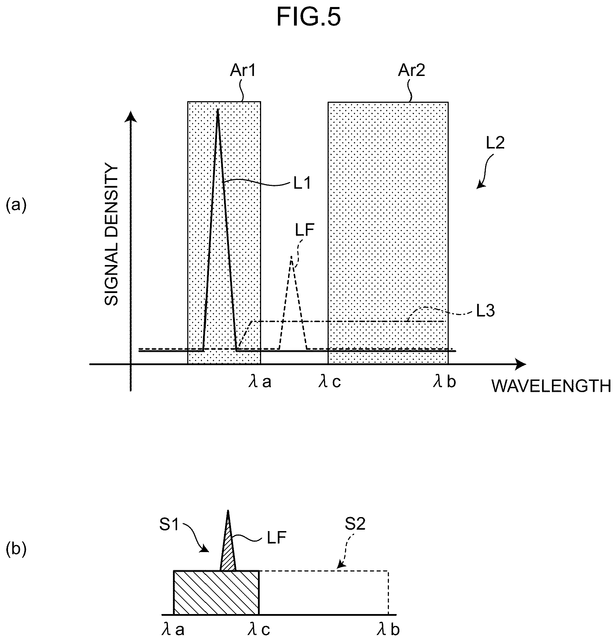

The filter 11 has a configuration in which a film of an inorganic material is optically formed on, for example, synthetic quartz. The synthetic quartz is a base material that emits a small amount of auto-fluorescent light. The filter 11 has a band-pass function, a shortpass function, or a long-pass function. Then, the filter 11 partially, substantially, or completely inhibits unnecessary light containing auto-fluorescent light L3 (see FIGS. 4 and 5) to be described later.

Note that details of the function of the filter 11 will be described later in "Function of Filter".

[Function of Filter]

Next, the function of the above-described filter 11 will be described.

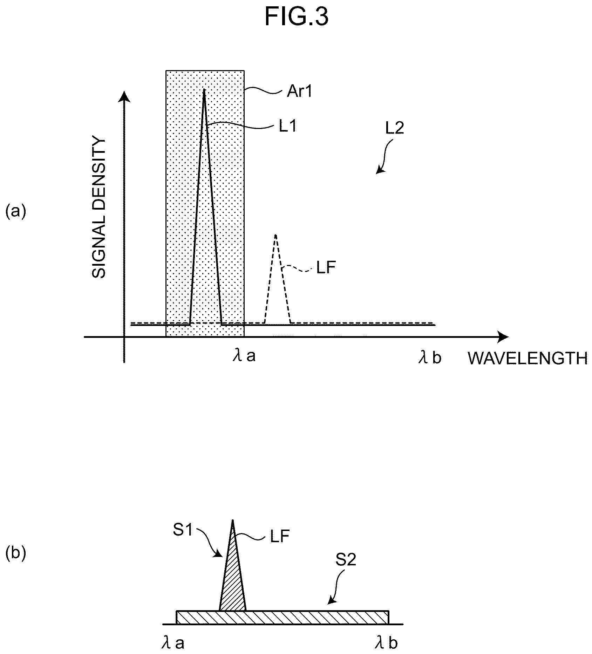

FIGS. 3 to 5 illustrate the function of the filter 11. Specifically, FIG. 3 illustrates an ideal state in which the auto-fluorescent light L3 serving as noise is not generated. FIG. 4 illustrates an actual state in which the auto-fluorescent light L3 serving as noise is generated. FIG. 5 illustrates the function of the filter 11. Note that (a) of FIG. 3, (a) of FIG. 4, and (a) of FIG. 5 illustrate the spectrum of second light L2. Furthermore, (b) of FIG. 3, (b) of FIG. 4, and (b) of FIG. 5 schematically illustrate a signal value S1 based on observation target fluorescent light LF and a signal value S2 based on unnecessary light containing the auto-fluorescent light L3. Note that, in FIGS. 3 to 5, the horizontal axes represent a wavelength [nm], and the vertical axes represent signal density [intensity/nm].

First, the ideal state in which the auto-fluorescent light L3 serving as noise is not generated will be described with reference to FIG. 3.

Excitation light L1 (FIG. 3) is applied from the light source device 3 to the observation target OB via a first optical path P1 (FIG. 1). The first optical path P1 follows a path of the light source device 3 to the light guide 4 to the insertion portion 2 to the observation target OB. When the excitation light L1 is applied to the observation target OB, a substance contained in the observation target OB is excited to emit the observation target fluorescent light LF (FIG. 3). The observation target fluorescent light LF is observation target light in fluorescent light observation. Then, the second light L2 (FIG. 3), which is return light of the excitation light L1 containing the observation target fluorescent light LF (FIG. 3), is propagated to the imaging element 513 via a second optical path P2 (FIG. 1). The second optical path P2 follows a path of the observation target OB to the insertion portion 2 to the imaging element 513. When propagated through the second optical path P2, the excitation light L1 contained in the second light L2 is partially, substantially, or completely inhibited by the excitation light cut filter 511. Note that the excitation light cut filter 511 performs cutting in a dotted wavelength band Ar1 in (a) of FIG. 3. Here, the "cuts" means that light is partially, substantially, or completely inhibited. "Cut" to be described below has similar meaning. Then, the imaging element 513 captures a subject image containing the observation target fluorescent light LF.

When a signal value based on the observation target fluorescent light LF is defined as S1 and a signal value based on a noise component is defined as S2, the S/N ratio of the signal value based on the observation target fluorescent light LF of a pixel signal obtained by imaging of the imaging unit 51 is S1/S2, which is a relatively large value as illustrated in (b) of FIG. 3.

Note that the signal values S1 and S2 are generated based on Expression (1) below. Here, in Expression (1), a wavelength band of wavelengths $\lambda a$ to $\lambda b$ includes the wavelength band of the observation target fluorescent light LF.
[Math 1]

$$Signal\ value$$

$$= \int_{\lambda a}^{\lambda b} (light\ spectrum\ that\ reaches\ imaging\ element) \qquad \cdots (1)$$

$$\times (wavelength\ sensitivity\ of\ imaging\ element)d\lambda$$

Next, the actual state in which the auto-fluorescent light L3 serving as noise is generated will be described with reference to FIG. 4.

When the excitation light L1 propagates through the first optical path P1 and when the second light L2 propagates through the second optical path P2, the auto-fluorescent light L3 (FIG. 4) is generated from a member forming an observation optical path P0 (FIG. 1) of the first and second optical paths P1 and P2 (hereinafter, referred to as auto-fluorescent light generating member) by applying the excitation light L1 and the second light L2 to the auto-fluorescent light generating member. Examples of the auto-fluorescent light generating member can include a component contained in a multicomponent glass such as a lens, a material component contained in a color filter, an adhesive that joins lenses or other optical members, and oil attached to an optical member such as a lens. As illustrated in FIG. 4, the auto-fluorescent light L3 has a wavelength band including the wavelength band of the observation target fluorescent light LF, which is observation target light in fluorescent light observation, and serves as noise in observing fluorescent light. Note that the auto-fluorescent light L3 is generated also by applying the excitation light L1 to the observation target OB.

That is, as illustrated in (b) of FIG. 4, the value of the S/N ratio (S1/S2) of the signal value based on the observation target fluorescent light LF of the pixel signal obtained by imaging of the imaging unit 51 is smaller than the value illustrated above in (b) of FIG. 3 since the signal value S2 based on a noise component containing the auto-fluorescent light L3 increases.

In particular, when the observation target fluorescent light LF from the observation target OB is faint, it is necessary to adjust the signal value based on the observation target fluorescent light LF imaged by the imaging element 513 for separating the observation target fluorescent light LF from the above-described auto-fluorescent light L3 and observing fluorescent light well. Adjusting the signal value based on the observation target fluorescent light LF is, however, difficult since (1) to (4) below have influences.

(1) Medical Agent

In general, a type and a dosage of a medical agent varies an amount of the observation target fluorescent light LF emitted from the medical agent.

The type of a medical agent to be administered to the observation target OB is selected in accordance with the observation target OB (e.g., cancer, blood, and lymph). Moreover, a medical agent capable of separating the wavelength of excitation light that excites a medical agent from the wavelength of the observation target fluorescent light LF emitted from the medical agent is selected in order to image the observation target fluorescent light LF emitted from the medical agent in the medical observation system 1. Medical agents selected as described above emit different amounts of observation target fluorescent light LF.

Furthermore, although the amount of the observation target fluorescent light LF can be adjusted by the dosage of a medical agent, unnecessarily increasing the dosage is difficult for achieving minimally invasive treatment.

That is, it is difficult to adjust the amount of the observation target fluorescent light LF by selecting the type of a medical agent or adjusting a dosage. As a result, it is difficult to adjust the signal value based on the observation target fluorescent light LF imaged by the imaging element 513 by selecting the type of a medical agent or adjusting a dosage.

(2) Observation Target

The amount of the observation target fluorescent light LF emitted from the observation target OB varies depending on the part/state of the observation target OB.

Specifically, the amount of the observation target fluorescent light LF emitted from an observation target OB in a part/state in which a medical agent easily stagnates increases. In contrast, the amount of the observation target fluorescent light LF emitted from an observation target OB in a part/state in which a medical agent easily flows and is difficult to stagnate decreases. Afterglow time is also shortened. Furthermore, when the observation target OB is a tumor, the spread, size, and depth thereof change the amount of the observation target fluorescent light LF received by the imaging element 513.

That is, it is difficult to adjust the amount of the observation target fluorescent light LF depending on the type or state of the observation target OB. As a result, it is difficult to adjust the signal value based on the observation target fluorescent light LF imaged by the imaging element 513 depending on the type or state of the observation target OB.

(3) Light Source Device

The amount of the excitation light L1 emitted from the light source device 3 varies the amount of the observation target fluorescent light LF.

In order to increase the amount of the excitation light L1, adjusting power to be supplied to the light source device 3 may be necessary. Power that can be supplied to the light source device 3 is, however, restricted in accordance with an upper limit value of power for operating the entire medical observation system 1. Furthermore, the amount of the excitation light L1 is required to be adjusted in consideration of heat generation in a member constituting an optical path of the excitation light L1 (e.g., heat generation between light guide 4 and insertion portion 2), an approach to a laser class, an amount of light energy received by the observation target OB or a surrounding living body (large amount of light energy may lead to burn), or speed of fading of the observation target fluorescent light LF emitted from a medical agent. Moreover, the amount of the excitation light L1 can be adjusted also by changing the number of light sources of the excitation light L1 mounted on the light source device 3. The number of light sources, however, may influence the size of the light source device 3. The size of the light source device 3 may be restricted by the size of a cart for carrying the light source device 3 and the like.

That is, it is difficult to adjust the amount of the observation target fluorescent light LF by adjusting the amount of the excitation light L1. As a result, it is difficult to adjust the signal value based on the observation target fluorescent light LF imaged by the imaging element 513 by adjusting the amount of the excitation light L1.

(4) Imaging Element

The signal value based on the observation target fluorescent light LF generated by the imaging element 513 varies depending on the amount of the observation target fluorescent light LF received by the imaging element 513.

In order to adjust the signal value based on the observation target fluorescent light LF, the imaging element 513 having sensitivity, configuration, and the like optimal to imaging of the observation target fluorescent light LF is desirably selected. The imaging element 513 may be, however, required not only to output an image for fluorescent light observation based on reception of the observation target fluorescent light LF in a predetermined wavelength band but to output an image for normal light observation based on reception of visible light such as white light. Furthermore, the imaging element 513 may also be required to output an image for fluorescent light observation in a wide wavelength band or a plurality of wavelength bands among wavelength bands containing visible light and invisible light. Moreover, even when the same medical agent is used, the amount of the observation target fluorescent light LF may change depending on a procedure or the observation target OB. The imaging element 513 may also be required to output an image for fluorescent light observation in accordance with a change in the amount of the observation target fluorescent light LF. In that case, the imaging element 513 needs to select an element that can address such observation. It may be impossible to use an imaging element having characteristics optimal to imaging of fluorescent light in a predetermined wavelength band. Furthermore, the imaging element 513 is disposed in the camera head 5. The camera head 5 has a size/weight suitable for observation, which may restrict the type including size of the imaging element 513. Note that not only in the configuration in which the imaging element 513 is disposed in the camera head 5 but in a case where the imaging element 513 is disposed at a distal end of a rigid endoscope or a flexible endoscope, there is a size/weight suitable for observation, which may restrict the type including size of the imaging element 513.

That is, it is difficult to adjust the signal value based on the observation target fluorescent light LF imaged by the imaging element 513 by selecting the type of the imaging element 513.

As described above, the signal value based on the observation target fluorescent light LF imaged by the imaging element 513 is determined under the above-described restrictions, so that the signal value cannot be easily adjusted.

Thus, in the first embodiment, the value of the S/N ratio (S1/S2) of the signal value based on the observation target fluorescent light LF of the pixel signal obtained by imaging of the imaging unit 51 is made more than 1 by providing the filter 11 between the eyepiece portion 21 and the camera head 5 on the second optical path P2 of the observation optical path P0. When the value of the S/N ratio (S1/S2) is more than 1, the control device 9 can generate an image, in which a user can easily identify a signal of the observation target fluorescent light LF and a signal of a noise component containing the auto-fluorescent light L3, by amplifying the S/N ratio by performing clamp processing, gain processing, gamma correction processing, or the like.

More specifically, the filter 11 cuts light in a wavelength band of wavelengths $\lambda c$ to $\lambda b$, excluding the wavelength

band of the observation target fluorescent light LF, in the wavelength band of the wavelengths $\lambda$a to $\lambda$b. That is, the filter 11 partially, substantially, or completely inhibits light other than the observation target fluorescent light LF, which is observation target light in fluorescent light observation. Note that the filter 11 performs cutting in a dotted wavelength band Ar2 in (a) of FIG. 5. The value of the S/N ratio (S1/S2) of the signal value of the pixel signal obtained by imaging of the imaging unit 51 is thus more than 1 since the signal value in the wavelength band of the wavelengths $\lambda$c to $\lambda$b in the signal value S2 based on the noise component containing the auto-fluorescent light L3 decreases ((b) of FIG. 5). An optical density (OD) value of a wavelength band in which cutting is performed in the filter 11 needs to be 1 or more, and is preferably 4 or more. Here, a higher OD value more enhances cutting effects, which enhances an image quality improvement effect. A film of a filter having a high OD value, however, needs to be formed in a dedicated facility, which easily increases cost. In a case of an OD value of approximately 4, a general-purpose film forming machine can perform production. Therefore, an OD value of 4 is preferably selected in practice in light of the balance between performance and cost.

Note that the value of the S/N ratio (S1/S2) may be made more than 1 by adjusting the OD value of a wavelength band in which cutting is performed in the filter 11 in the wavelength band of the wavelengths $\lambda$c to $\lambda$b. Furthermore, when the value of the S/N ratio (S1/S2) of the signal value of the pixel signal obtained by imaging of the imaging unit 51 is more than 1, the filter 11 may cut light in a partial wavelength band of the wavelength band of the observation target fluorescent light LF.

[Method of Calculating S/N Ratio]

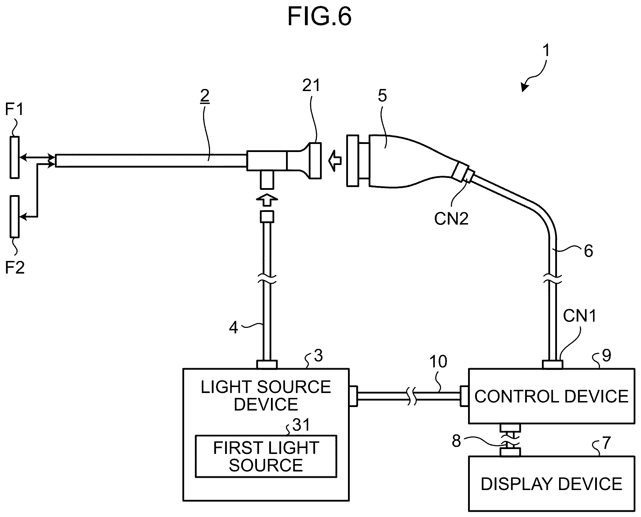

FIG. 6 illustrates a method of calculating an S/N ratio. Specifically, FIG. 6 corresponds to FIG. 1.

In a method of calculating an S/N ratio according to the embodiment, "2.D.8. Excitation light crosstalk" and "3.G. Excitation light crosstalk" in Title "Performance test methods for near-infrared fluorescence imaging" of Literature A below are referred to.

(Literature A)

"MEDICAL PHYSICS" Volume 47, Issue 8, August 2020, Pages 3389-3401

As illustrated in FIG. 6, first and second subjects (phantoms) F1 and F2 are used in the method of calculating an S/N ratio.

The first subject F1 is a subject (phantom) not containing a medical agent but containing only scattering particles.

The second subject F2 is a subject (phantom) containing a medical agent at a concentration appropriate for a living body desired to be observed.

Then, the S/N ratio is calculated by Expression (2) below.

(Math 2)

$$\text{S/N ratio} = ((c) - (a))/((b) - (a)) \qquad (2)$$

Here, (a) of Expression (2) represents a luminance level based on a pixel signal output from the imaging element 513 in a state in which no external light enters the camera head 5 and a state in which the first light source 31 is turned off in the medical observation system 1 in FIG. 6. Note that (a) may represent a luminance level based on a pixel signal output from the imaging element 513 in a state in which a mechanical shutter is disposed between the camera head 5 and the eyepiece portion 21 and the mechanical shutter prevents light from entering the camera head 5. Furthermore, (b) represents a luminance level based on a pixel signal output from the imaging element 513 in a case where the first light source 31 is turned on, the excitation light L1 is applied to the first subject F1, and the imaging element 513 images the second light L2 in the medical observation system 1 in FIG. 6. Moreover, (c) represents a luminance level based on a pixel signal output from the imaging element 513 in a case where the first light source 31 is turned on, the excitation light L1 is applied to the second subject F2, and the imaging element 513 images the second light L2 in the medical observation system 1 in FIG. 6.

The filter 11 according to the present disclosure adjusts a wavelength band in which cutting is performed and the OD value of the wavelength band such that an S/N ratio is more than 1. The S/N ratio is calculated by Expression (2) by using a pixel signal (RAW signal) output from the imaging element 513 before image processing is executed on the pixel signal. The filter 11 according to the present disclosure adjusts a wavelength band in which cutting is performed and the OD value of the wavelength band such that an S/N ratio is 4 or more. The S/N ratio is calculated by Expression (2) by using a pixel signal output from the imaging element 513 after image processing is executed on the pixel signal.

According to the above-described first embodiment, the following effects are exhibited.

The medical observation system 1 according to the first embodiment includes the filter 11 that is disposed on an observation optical path P0 and that partially, substantially, or completely inhibits unnecessary light containing auto-fluorescent light L3 generated from a member forming the observation optical path P0 by at least one of the excitation light L1 and the second light L2. Then, the filter 11 makes the value of the S/N ratio (S1/S2) of a signal value based on the observation target fluorescent light LF of a pixel signal obtained by imaging of the imaging unit 51 more than 1. Therefore, the control device 9 can generate an image, in which a user can easily identify a signal of the observation target fluorescent light LF and a signal of a noise component containing the auto-fluorescent light L3.

Therefore, the medical observation system 1 according to the first embodiment can observe fluorescent light well.

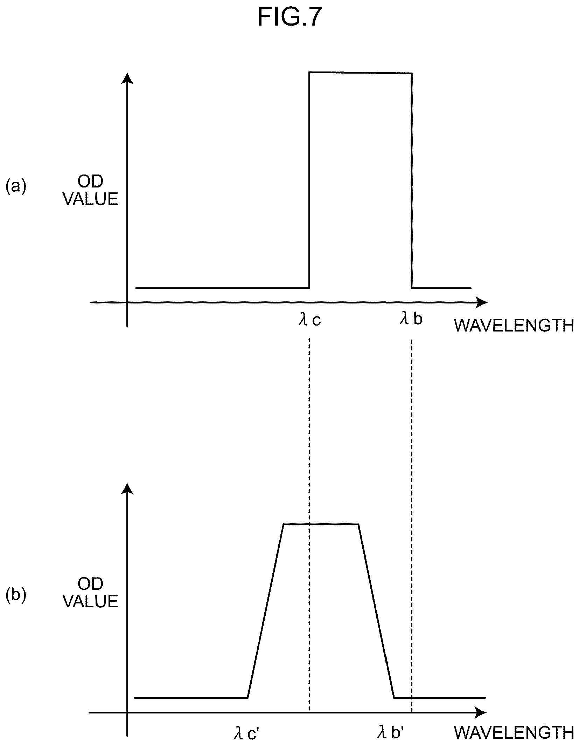

FIG. 7 illustrates effects of the first embodiment. Specifically, (a) of FIG. 7 illustrates transmission characteristics (filter characteristics) of the filter 11 in a case where the filter 11 is disposed at a specific position (between eyepiece portion 21 and camera head 5). In contrast, (b) of FIG. 7 illustrates transmission characteristics (filter characteristics) of the filter 11 in a case where the filter 11 is disposed at a position other than the specific position. Note that, in FIG. 7, the horizontal axis represents a wavelength [nm], and the vertical axis represents an OD value.

The transmission characteristics (filter characteristics) of the filter 11 change depending on an incident angle of light.

Light emitted from the eyepiece portion 21 is substantially parallel light. Therefore, when the filter 11 is disposed between the eyepiece portion 21 and the camera head 5, the second light L2 is incident on the filter 11 as substantially parallel light. When the second light L2 is incident as substantially parallel light as described above, the filter 11 has transmission characteristics of a design value as illustrated in (a) of FIG. 7.

In contrast, in a case of light beam distribution of NA 0.2 or more (light beam of ±12 degrees or more), as illustrated in (b) of FIG. 7, the filter 11 may have transmission characteristics deteriorated from the transmission characteristics of the design value. Specifically, a wavelength band in which cutting is performed shifts to the short wavelength band, so that the wavelength band in which cutting is performed may be no longer the optimal wavelength band. Furthermore, rise and fall of the OD value in the filter characteristics may change from sharp rise and fall ((a) of FIG. 7) in design to gentle rise and fall ((b) of FIG. 7). Moreover, the OD value of the wavelength band in which cutting is performed may decrease.

As described above, the transmission characteristics of the filter 11 can be maintained at the transmission characteristics of a design value, and unnecessary light containing the auto-fluorescent light L3 can be partially, substantially, or completely inhibited at the design value by arranging the filter 11 between the eyepiece portion 21 and the camera head 5.

Furthermore, the filter 11 may be detachably connected to the eyepiece portion 21.

In such a detachable configuration, a plurality of filters 11 is prepared in accordance with an observation target substance such as a medical agent and a fluorescent dye to be imparted to the observation target OB. The plurality of filters 11 has different wavelength bands in which cutting is desired to be performed and different levels of an amount of light desired to be cut. The filter 11 can be selected and used in accordance with the observation target substance such as the medical agent and the fluorescent dye to be used. Note that, as the filter 11, a filter that addresses a plurality of observation target substances (e.g., medical agent and other medical agent, and medical agent and auto-fluorescent light of subject) required to be simultaneously observed may be used. A filter that addresses a single observation target substance (medical agent and auto-fluorescent light of subject) may be used. A plurality of filters having the same or different wavelength bands in which cutting is desired to be performed or having the same or different levels of an amount of light desired to be cut may be superimposed on the eyepiece portion 21.

Then, the filter 11 according to the present disclosure can observe fluorescent light well in first to sixth scenes to be described below. Note that, although, in the first to sixth scenes to be described below, ICG is adopted as a medical agent to be used, fluorescent light can be observed well as in a scene in which another medical agent is used.

[First Scene]

Examples of a scene in which the observation target fluorescent light LF is faint in a case where ICG is used include a scene in which a pediatric patient has surgery.

A dose of ICG needs to be set in accordance with the weight of a patient. Therefore, when ICG is administered to a pediatric patient, the dose of ICG needs to be decreased in proportion to the weight as compared to that for an adult patient. That is, when ICG is administered to a pediatric patient, the ICG concentration may be low, and the observation target fluorescent light LF may be faint.

Then, in surgery on a pediatric patient, even when the observation target fluorescent light LF is faint, the filter 11 can partially, substantially, or completely inhibit unnecessary light containing the auto-fluorescent light L3, and fluorescent light can be observed well.

[Second Scene]

Examples of the scene in which the observation target fluorescent light LF is faint in a case where ICG is used include a scene in which an endobronchial injection method is performed.

The endobronchial injection method is an approach of identifying a segment in lung segmental excision. Specifically, in the endobronchial injection method, ICG is sprayed to a segmental bronchus with a bronchoscope, and a lung segment to be excised is identified by fluorescent light observation. That is, in the endobronchial injection method, increasing the ICG concentration may be difficult, and the observation target fluorescent light LF may be faint as compared to a case where a lung segment to be excised is identified by a method of intravenously injecting ICG (hereinafter, referred to as ICG intravenous injection method).

Then, in the endobronchial injection method, even when the observation target fluorescent light LF is faint, the filter 11 can partially, substantially, or completely inhibit unnecessary light containing the auto-fluorescent light L3, and fluorescent light can be observed well.

[Third Scene]

Examples of the scene in which the observation target fluorescent light LF is faint in a case where ICG is used include a scene in which a small-diameter endoscope obtained by decreasing the outer diameter of the insertion portion 2 is used.

As fluorescent light observation using ICG for the lower digestive tract becomes common, there is a growing need for still minimally invasive surgery. Then, under a condition with a spatial restriction as in trans-anal total mesorectal excision and single port surgery, a small-diameter endoscope is often used to enhance the operability of forceps. In the small-diameter endoscope, however, a light guide path for excitation light is narrowed as compared to that in a large-diameter endoscope obtained by increasing the outer diameter of a normal endoscope or the insertion portion 2, so that the energy of the excitation light to be emitted decreases, and the observation target fluorescent light LF may be faint.

Then, even when a small-diameter endoscope is used and the observation target fluorescent light LF is faint, the filter 11 can partially, substantially, or completely inhibit unnecessary light containing the auto-fluorescent light L3, and fluorescent light can be observed well.

[Fourth Scene]

Examples of the scene in which the observation target fluorescent light LF is faint in a case where ICG is used include a scene in which a low concentration of ICG is used, for example, at the first time of a plurality of times at the time when a lung segment is identified by the plurality of times of fluorescent light observation by the ICG intravenous injection method.

A problem of the ICG intravenous injection method is that follow-up observation is difficult since ICG intravenously injected once is carried by the bloodstream, remains, and continues to glow until ICG is metabolized in the liver. For example, when a segment other than an observation target such as the whole lung glows due to stopping of bleeding at an erroneous portion and loose ligation in segmental excision of a lobe of the lung, identifying the segment again by the ICG intravenous injection method is difficult for the above-described reason. Furthermore, since an upper limit is set to a dose of ICG to an adult, it is difficult to identify a segment by the ICG intravenous injection method again after the ICG is metabolized.

Thus, when a lung segment is identified at the first time, ICG is intravenously injected at a low ICG concentration (e.g., concentration of 1/10 of normal concentration) to observe fluorescent light. When an appropriate segment cannot be identified by the fluorescent light observation, ICG is intravenously injected again at a normal ICG concentration to observe fluorescent light. When a lung segment is identified at the first time, the ICG concentration is set to be low, so that the observation target fluorescent light LF may be faint as compared to that at a normal ICG concentration.

Then, even when the observation target fluorescent light LF is faint in observing fluorescent light at a low ICG concentration, the filter 11 can partially, substantially, or completely inhibit unnecessary light containing the auto-fluorescent light L3, and fluorescent light can be observed well. Thereafter, fluorescent light is observed at a normal ICG concentration. A lung segment can be identified with higher accuracy by observing fluorescent light a plurality of times in the same part as described above.

[Fifth Scene]

Examples of the scene in which the observation target fluorescent light LF is faint in a case where ICG is used include a scene in which a low concentration of ICG is used, for example, at the first time of a plurality of times of fluorescent light observation at the time when the bloodstream in intestinal anastomotic failure is checked by a plurality of times of fluorescent light observation by the ICG intravenous injection method.

When the bloodstream in intestinal anastomotic failure is checked by fluorescent light observation, and when the bloodstream cannot be appropriately checked due to a device setting mistake, a subject buried in a tissue such as fat, and the like, ICG metabolization takes time. It is thus difficult to check the bloodstream by the ICG injection method again.

Thus, when the bloodstream is checked at the first time as in the above-described fourth scene, ICG is intravenously injected at a low ICG concentration (e.g., concentration of 1/10 of normal concentration) to observe fluorescent light. When the bloodstream cannot be appropriately checked by the fluorescent light observation, ICG is intravenously injected again at a normal ICG concentration to observe fluorescent light. When the bloodstream is checked at the first time, the ICG concentration is set to be low, so that the observation target fluorescent light LF may be faint as compared to that at a normal ICG concentration.

Then, even when the observation target fluorescent light LF is faint in observing fluorescent light at a low ICG concentration, the filter 11 can partially, substantially, or completely inhibit unnecessary light containing the auto-fluorescent light L3, and fluorescent light can be observed well. Thereafter, fluorescent light is observed at a normal ICG concentration. Complications such as peritonitis due to anastomotic failure can be reduced by observing fluorescent light a plurality of times in the same part as described above.

[Sixth Scene]

Examples of the scene in which the observation target fluorescent light LF is faint in a case where ICG is used include a scene in which a fluorescent image is fluorescently observed by using a 3D image (hereinafter, referred to as 3D fluorescent light observation).

When the 3D fluorescent light observation is performed, a scope of a twin-lens relay method may be used as the insertion portion 2. In the scope of the twin-lens relay method, two optical paths are arranged in parallel in the scope. Furthermore, optical systems are arranged in the two optical paths. Then, the scope of the twin-lens relay method captures observation light for right and left eyes having parallax with each other with the two optical systems, and guides the light to the imaging element 513 (e.g., see JP H6-160731 A). In such a scope of the twin-lens relay method, as in the small-diameter endoscope described above in the third scene, a light guide path of excitation light is narrowed by the two optical paths as compared to that in a normal endoscope, so that the energy of the excitation light to be emitted decreases, and the observation target fluorescent light LF may be faint.

Furthermore, when 3D fluorescent light observation is performed, two imaging elements that image observation light for right and left eyes may be used as imaging elements 513. When two imaging elements are used as the imaging elements 513 as described above, the size decreases as compared to that in a case where one imaging element is used, so that light receiving energy of the observation target fluorescent light LF may decrease, and a signal value based on the observation target fluorescent light LF may be faint.

Note that, although a case where a rigid endoscope for 3D fluorescent light observation is used has been exemplified above, the observation target fluorescent light LF may be faint, and a signal value based on the observation target fluorescent light LF may be faint as in a case where a flexible endoscope for 3D fluorescent light observation is used.

Then, even when the observation target fluorescent light LF is faint in surgery using an endoscope for 3D fluorescent light observation, the filter 11 can partially, substantially, or completely inhibit unnecessary light containing the auto-fluorescent light L3, and 3D fluorescent light observation can be performed well.

(Second Embodiment)

Next, a second embodiment will be described.

The same reference signs are attached below to configurations similar to those in the above-described first embodiment, and detailed description thereof will be omitted or simplified.

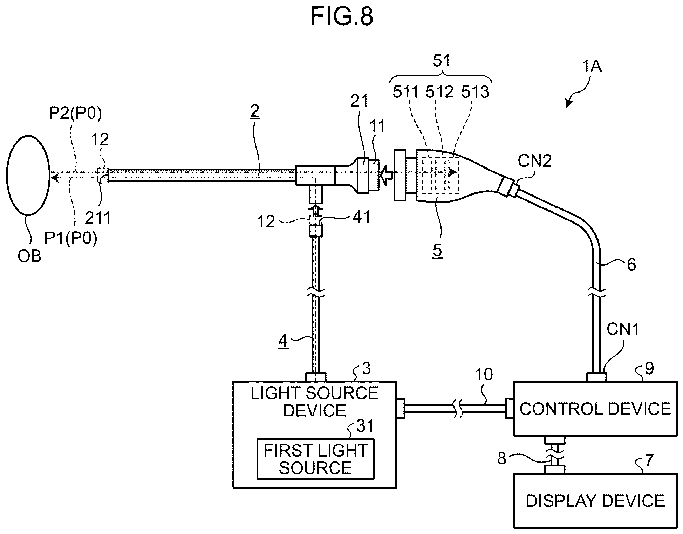

FIG. 8 illustrates a configuration of a medical observation system 1A according to the second embodiment. Specifically, FIG. 8 corresponds to FIG. 1.

In the medical observation system 1A according to the second embodiment, as illustrated in FIG. 8, a filter 12 is adopted in addition to the filter 11 as a filter that partially, substantially, or completely inhibits unnecessary light containing the auto-fluorescent light L3. Note that, for convenience of description, the filter 11 will be hereinafter referred to as a first filter 11, and the filter 12 will be hereinafter referred to as a second filter 12.

As illustrated in FIG. 8, the second filter 12 is disposed, on the first optical path P1, between the light guide 4 and the insertion portion 2 (emission end 41 of light guide 4) or at an emission end 211, which emits the excitation light L1, of the insertion portion 2. Note that, for convenience of description, FIG. 8 illustrates the second filter 12 at two positions of the emission end 41 of the light guide 4 and the emission end 211 of the insertion portion 2. Then, the second filter 12 partially, substantially, or completely inhibits light other than the excitation light L1. More specifically, when the excitation light L1 has a short wavelength (hereinafter, referred to as case of short-wave excitation), the second filter 12 is disposed at the emission end 211 of the insertion portion 2. Examples of light having the short wavelength can include visible light and ultraviolet light. In contrast, when the excitation light L1 has a long wavelength (hereinafter, referred to as case of long-wave excitation), the second filter 12 is disposed at the emission end 41 of the light guide 4. Examples of light having the long wavelength can include infrared light.

The difference in effects depending on the arrangement position of the second filter 12 between the case of short-wave excitation and the case of long-wave excitation will be described below.

First, the case of short-wave excitation will be described.

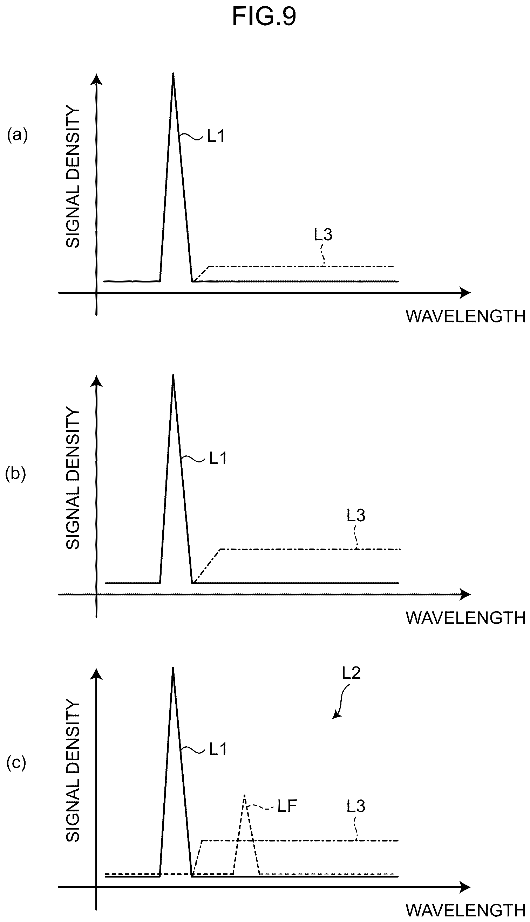

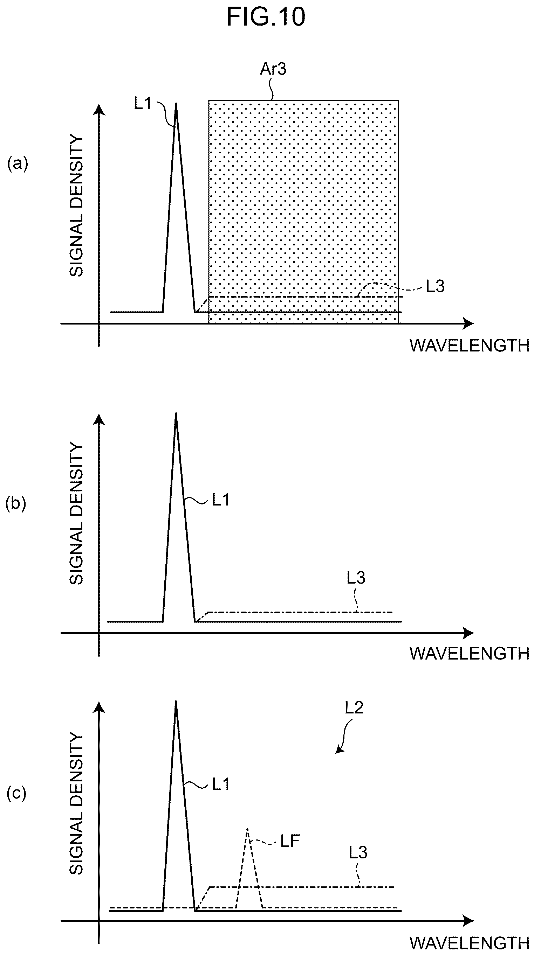

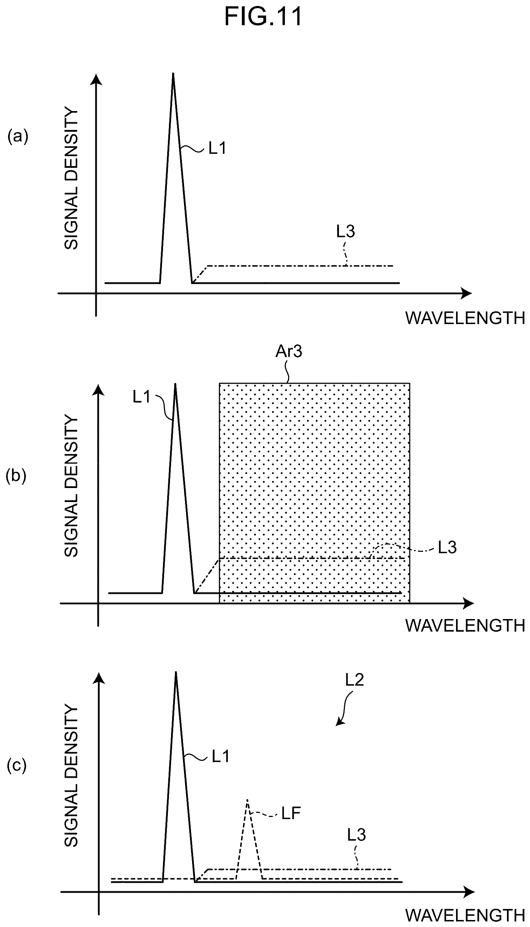

FIGS. 9 to 11 illustrate the function of the second filter 12 in the case of short-wave excitation. Specifically, FIG. 9 illustrates a spectrum of light immediately before being emitted from the emission end 41 of the light guide 4 in a case where the second filter 12 is not provided ((a) of FIG. 9), a spectrum of light immediately before being emitted from the emission end 211 of the insertion portion 2 ((b) of FIG. 9), and a spectrum of return light immediately before being captured in the insertion portion 2 from the observation target OB ((c) of FIG. 9). FIG. 10 illustrates a spectrum of light immediately before being emitted from the emission end 41 in a case where the second filter 12 is disposed at the emission end 41 of the light guide 4 ((a) of FIG. 10), a spectrum of light immediately before being emitted from the emission end 211 of the insertion portion 2 ((b) of FIG. 10), and a spectrum of return light immediately before being captured in the insertion portion 2 from the observation target OB ((c) of FIG. 10). FIG. 11 illustrates a spectrum of light immediately before being emitted from the emission end 41 of the light guide 4 in a case where the second filter 12 is disposed at the emission end 211 of the insertion portion 2 ((a) of FIG. 11), a spectrum of light immediately before being emitted from the emission end 211 ((b) of FIG. 11), and a spectrum of return light immediately before being captured in the insertion portion 2 from the observation target OB ((c) of FIG. 11). Note that the second filter 12 performs cutting in a dotted wavelength band Ar3 in (a) of FIG. 10 and (b) of FIG. 11. Note that, in FIGS. 9 to 11, the horizontal axes represent a wavelength [nm], and the vertical axes represent signal density [intensity/nm].

As illustrated in (a) of FIG. 9, (a) of FIG. 10, and (a) of FIG. 11, when the excitation light L1 propagates through the light guide 4, the auto-fluorescent light L3 is generated from an auto-fluorescent light generating member included in the light guide 4.

Furthermore, as illustrated in (b) of FIG. 9 and (b) of FIG. 11, also when the excitation light L1 propagates through the insertion portion 2, the auto-fluorescent light L3 is generated from an auto-fluorescent light generating member included in the insertion portion 2. Therefore, when the second filter 12 is not provided or the second filter 12 is disposed at the emission end 211 of the insertion portion 2, the intensity of the auto-fluorescent light L3 increases immediately before light is emitted from the emission end 211 of the insertion portion 2 as compared to that in a stage immediately before light is emitted from the emission end 41 of the light guide 4.

In contrast, when the second filter 12 is disposed at the emission end 41 of the light guide 4, the second filter 12 partially, substantially, or completely inhibits the auto-fluorescent light L3 generated from the auto-fluorescent light generating member included in the light guide 4. Therefore, as illustrated in (b) of FIG. 10, the auto-fluorescent light L3 contained in light immediately before being emitted from the emission end 211 of the insertion portion 2 has an intensity lower than those in (b) of FIG. 9 and (b) of FIG. 11.

Moreover, as illustrated in (c) of FIG. 9 and (c) of FIG. 10, also when the excitation light L1 is applied to the observation target OB, the auto-fluorescent light L3 may be generated from the observation target OB. In that case, the intensity of the auto-fluorescent light L3 increases immediately before return light is captured in the emission end 211 of the insertion portion 2 as compared to that in a stage immediately before light is emitted from the emission end 211 of the insertion portion 2.

In contrast, when the second filter 12 is disposed at the emission end 211 of the insertion portion 2, the second filter 12 partially, substantially, or completely inhibits the auto-fluorescent light L3 generated from both the auto-fluorescent light generating member included in the light guide 4 and the auto-fluorescent light generating member included in the insertion portion 2. Therefore, as illustrated in (c) of FIG. 11, the auto-fluorescent light L3 contained in return light immediately before being captured in the emission end 211 of the insertion portion 2 has an intensity lower than those in (c) of FIG. 9 and (c) of FIG. 11.

In the case of short-wave excitation, a large amount of auto-fluorescent light L3 is generated since the excitation light L1 applied to the auto-fluorescent light generating member has a large amount of energy. Then, as can be seen from comparison between (c) of FIG. 10 and (c) of FIG. 11, providing the second filter 12 in a further rear stage of the optical path on the first optical path P1 is effective. Therefore, in the case of short-wave excitation, the second filter 12 is disposed at the emission end 211 of the insertion portion 2.

Next, the case of long-wave excitation will be described.

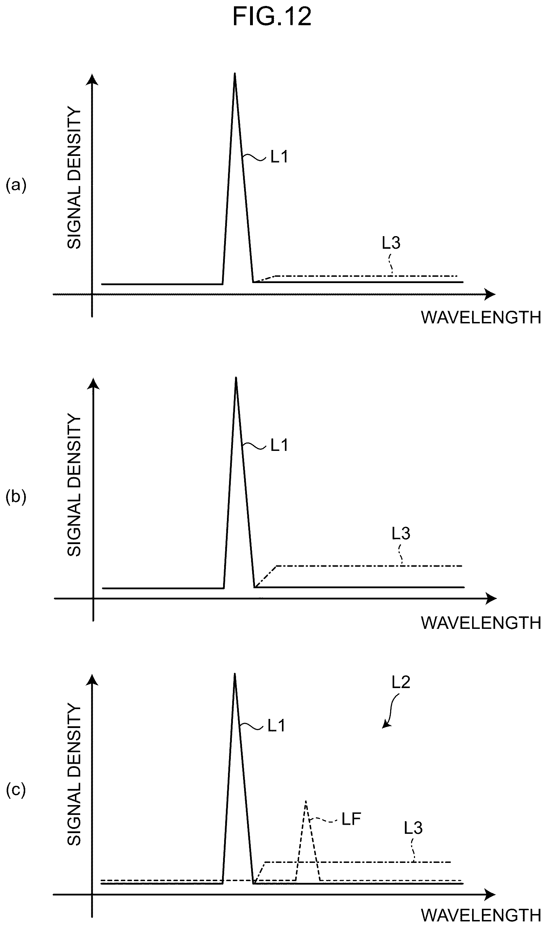

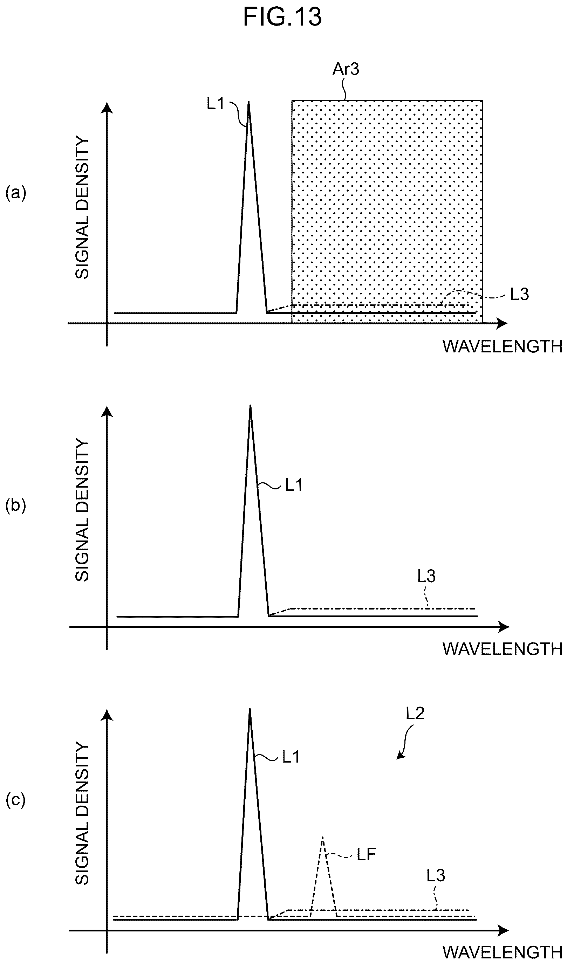

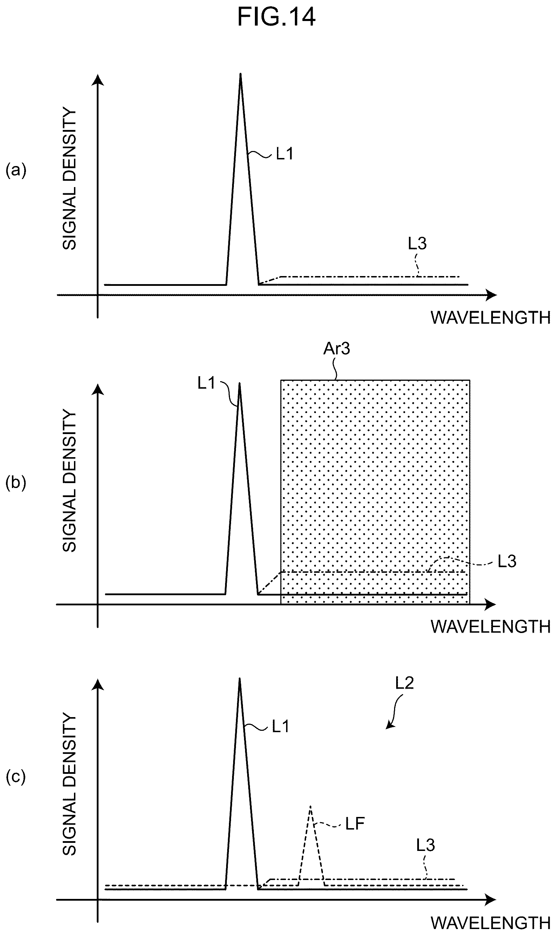

FIGS. 12 to 14 illustrate the function of the second filter 12 in the case of long-wave excitation. Specifically, FIG. 12 illustrates the case where the second filter 12 is not provided, and corresponds to FIG. 9. FIG. 13 illustrates the case where the second filter 12 is disposed at the emission end 41 of the light guide 4, and corresponds to FIG. 10. FIG. 14 illustrates the case where the second filter 12 is disposed at the emission end 211 of the insertion portion 2, and corresponds to FIG. 11. Note that, in FIGS. 12 to 14, the horizontal axes represent a wavelength [nm], and the vertical axes represent signal density [intensity/nm].

Also in the case of long-wave excitation, as illustrated in FIGS. 12 to 14, the behavior of the auto-fluorescent light L3 is similar to that in the case of short-wave excitation described above.

In the case of long-wave excitation, as can be seen from comparison between FIGS. 12 to 14 and FIGS. 9 to 11, a relatively low amount of auto-fluorescent light L3 is generated since the excitation light L1 applied to the auto-fluorescent light generating member has a small amount of energy. Then, as can be seen from comparison between (c) of FIG. 13 and (c) of FIG. 14, the auto-fluorescent light L3 contained in return light immediately before being captured in the emission end 211 of the insertion portion 2 has substantially the same intensity in the case where the second filter 12 is provided at the emission end 41 of the light guide 4 and in the case where the second filter 12 is provided at the emission end 211 of the insertion portion 2. Here, the emission end 211 of the insertion portion 2 is a part to be inserted into the observation target OB (living body), and is relatively difficult to be designed. Therefore, in the case of long-wave excitation, the second filter 12 is disposed at the emission end 41 of the light guide 4.

According to the above-described second embodiment, the following effects are exhibited in addition to the effects similar to those in the first embodiment as described above.

In the medical observation system 1 according to the second embodiment, the first and second filters 11 and 12 are mounted, so that unnecessary light containing the auto-fluorescent light L3 can be partially, substantially, or completely inhibited effectively.

Furthermore, the second filter 12 may be fixed to the emission end 41 of the light guide 4 or the emission end 211 of the insertion portion 2. In this case, a component is not added, so that a user can perform handling more easily than in a configuration in which the first filter 11 is detachable.

(Other Embodiments)

Although the embodiments for carrying out the present disclosure have been described so far, the present disclosure should not be limited only by the above-described embodiments.

In the above-described first and second embodiments, not only one or two but three or more filters according to the present disclosure may be provided. Furthermore, the arrangement positions of the filters according to the present disclosure are not limited to the arrangement positions described in the above-described first and second embodiments as long as the arrangement positions are provided on the observation optical path P0. The filters according to the present disclosure may be disposed in, for example, the light source device 3, the light guide 4, the insertion portion 2, or the camera head 5 (e.g., imaging surface of imaging element 513) as long as the filters are located on the observation optical path PO. Moreover, the filters according to the present disclosure may be disposable. Furthermore, neutral density (ND) filters may be adopted as the filters according to the present disclosure.

Although, in the above-described first and second embodiments, the excitation light L1 has been adopted as the first light according to the present disclosure, this is not a limitation. In addition to the excitation light L1, normal observation light such as visible light (broadband light such as white light and narrowband light such as red light, green light, and blue light) may be adopted as the first light according to the present disclosure. Examples of the configuration of a light source device that emits the normal observation light can include a configuration including a white LED and a configuration including three light sources and an optical element. Each of the three light sources emit red light, green light, or blue light. The optical element synthesizes the red light, the green light, and the blue light.

Furthermore, when two of the excitation light L1 and the normal observation light are adopted as the first light according to the present disclosure, one or two imaging elements 513 may be provided, or more imaging elements 513 may be provided.

For example, when only one imaging element 513 is provided, the excitation light L1 and the normal observation light are made to be emitted by time division. The imaging element 513 images, by time division, the excitation light L1 and the normal observation light at the timing when the excitation light L1 is emitted and the timing when the observation light is emitted.

Furthermore, for example, when two imaging elements 513 are provided, a color separation optical device such as a prism is adopted. Specifically, the color separation optical device is disposed on the second optical path P2, and separates return light of the normal observation light from the observation target OB propagated along the second optical path P2 from return light (observation target fluorescent light LF) of the excitation light L1 from the observation target OB. Then, one of the two imaging elements 513 images the return light of the normal observation light. The other imaging element 513 images return light (observation target fluorescent light LF) of the excitation light L1. Note that, when the color separation optical device is adopted, a filter according to the present disclosure may be disposed in the color separation optical device.

As described above, when two of the excitation light L1 and the normal observation light are adopted as the first light according to the present disclosure, a filter according to the present disclosure needs a configuration in which light excluding return light of normal observation light from a subject and the observation target fluorescent light LF is cut.

Note that a case where two of the excitation light L1 and the normal observation light are adopted as the first light according to the present disclosure has been described above. Even when only one of the excitation light L1 or only one of the normal observation light is adopted as the first light according to the present disclosure, one or two or more imaging elements 513 may be provided.

Variations 1 to 3 below may be adopted in the above-described first and second embodiments.

(Variation 1)

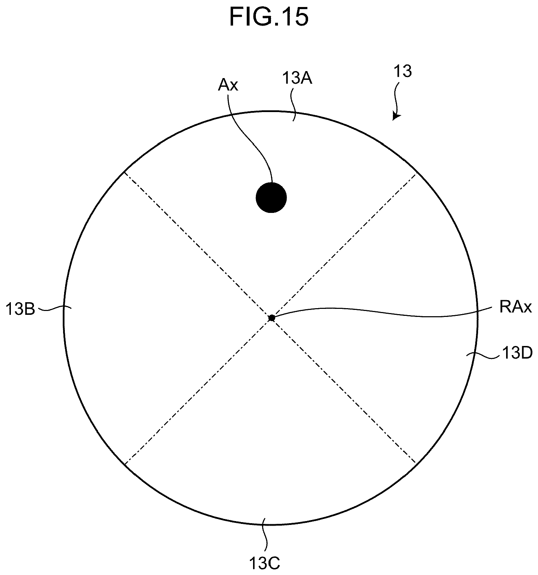

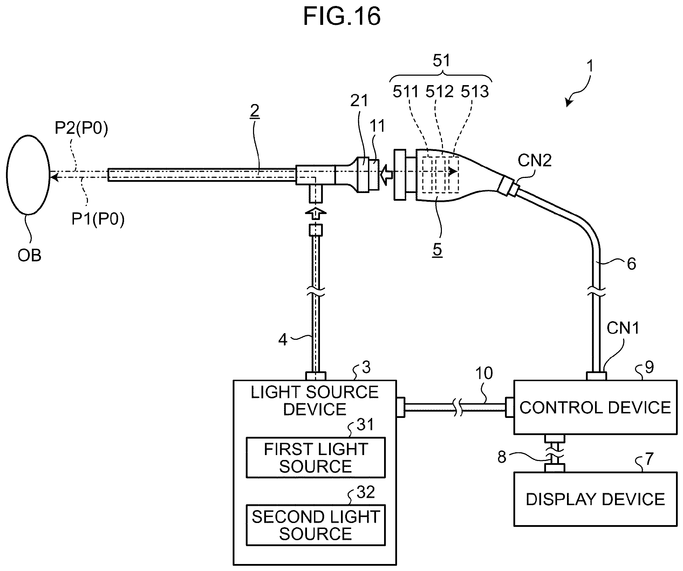

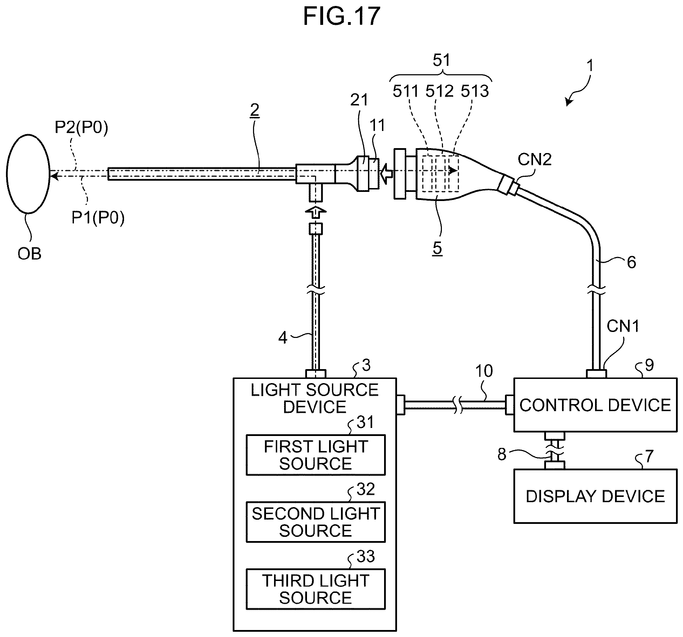

FIGS. 15 to 17 illustrate Variation 1 of the first and second embodiments. Specifically, FIG. 15 illustrates the shape of a filter 13 according to Variation 1. The filter 13 is viewed along an optical axis Ax of light incident on the filter 13. FIGS. 16 and 17 correspond to FIG. 1.

Although the filter 11 described above in the first and second embodiments has the same transmission characteristics throughout the whole surface, this is not a limitation.

As illustrated in FIG. 15, the filter 13 according to Variation 1 has a wheel shape that enables rotation around a rotation axis RAx, and is divided into a plurality of regions 13A to 13D having different transmission characteristics. Note that, in Variation 1, the four regions 13A to 13D are obtained by the division, another number of regions may be obtained as long as two or more regions are obtained. The transmission characteristics of the four regions 13A to 13D are set in accordance with a medical agent or a fluorescent dye to be imparted to the observation target OB. Then, the filter 13 is rotated such that a region corresponding to a medical agent or a fluorescent dye imparted to the observation target OB among the four regions 13A to 13D is located at the optical axis Ax. Then, the region partially, substantially, or completely inhibits light other than the observation target fluorescent light LF in accordance with a medical agent and a fluorescent dye imparted to the observation target OB.

According to Variation 1 described above, the following effect is exhibited in addition to effects similar to those in the above-described first and second embodiments.

The filter 13 according to Variation 1 has a wheel shape that enables rotation around the rotation axis RAx, and has the above-described plurality of regions 13A to 13D.

Therefore, one filter 13 can address various medical agents and fluorescent dyes, and can improve convenience.

Note that the configuration of the filter 13 is not limited to the above-described configuration. For example, the four regions 13A to 13D may have different OD values.

Furthermore, the filter 13 according to Variation 1 can be applied to, for example, surgery using a plurality of medical agents illustrated in Table 1 below. Note that, in Table 1, "∘" is attached to a medical agent to be used, and "×" is attached to a medical agent not to be used.

**Table 1**

| Category | Target disease | Medical agent | | | | |
| --- | --- | --- | --- | --- | --- | --- |
| | | Fluorescein | AL-M-488 | LU-M-015 | 5-ALA | ICG |
| Surgery | Lung cancer | × | ○ | × | × | ○ |
| | Colorectal cancer | | | | | |
| | Childhood brain tumor | | | | | |
| | Breast cancer | | | ○ | | |
| Photo dynamic diagnosis | Brain tumor treatment | | | × | ○ | × |
| | Superficial cell cancer on urinary tract | ○ | × | | | |

Here, when the excitation light L1 in a wavelength band of approximately 470 to 480 [nm] is applied, fluorescein emits the observation target fluorescent light LF of approximately 520 [nm]. Furthermore, when the excitation light L1 in a wavelength band of approximately 488 [nm] is applied, ALM-488 emits the observation target fluorescent light LF in a wavelength band of approximately 530 [nm]. Moreover, when the excitation light L1 in a wavelength band of approximately 650 [nm] is applied, LUM-015 emits the observation target fluorescent light LF in a wavelength band of approximately 675 [nm]. Furthermore, when the excitation light L1 in a wavelength band of approximately 405 [nm] is applied, 5-ALA emits the observation target fluorescent light LF in a wavelength band of approximately 530 to 630 [nm]. Moreover, when the excitation light L1 in a wavelength band of approximately 805 [nm] is applied, ICG emits the observation target fluorescent light LF in a wavelength band of approximately 830 [nm].

For example, when lymph node dissection is performed in lobectomy for treating lung cancer, ALM-488 and ICG are used as illustrated in Table 1. That is, ALM-488 visualizes a left recurrent laryngeal nerve. ICG visualizes a lymph node. The left recurrent laryngeal nerve and the lymph node are identified by visualizing the left recurrent laryngeal nerve and the lymph node as described above, so that recurrent laryngeal nerve paralysis in the lobectomy can be reduced. In the case, since two medical agents of ALM-488 and ICG are used, the medical observation system 1 needs a second light source 32 in addition to the first light source 31 as illustrated in FIG. 16. The first light source 31 emits the excitation light L1 that excites one of the two medical agents. The second light source 32 emits the excitation light L1 that excites the other medical agent. Note that the first and second light sources 31 and 32 correspond to the first light source according to the present disclosure. Furthermore, when one medical agent that makes the observation target fluorescent light LF faint among the two medical agents of ALM-488 and ICG is used, the medical observation system 1 positions one of the plurality of regions 13A to 13D of the filter 13 on the optical axis Ax (hereinafter, referred to as first mode). When the other medical agent is used, the medical observation system 1 positions another region of the plurality of regions 13A to 13D on the optical axis Ax (hereinafter, referred to as second mode). Then, a doctor who performs the lobectomy can grasp the position of one of the left recurrent laryngeal nerve and the lymph node from an image displayed on the display device 7 in the first mode, and grasp the position of the other of the left recurrent laryngeal nerve and the lymph node from an image displayed on the display device 7 in the second mode.

Furthermore, for example, when lateral side dissection is performed in rectectomy for treating colorectal cancer, ALM-488 and ICG are used as illustrated in Table 1. That is, ALM-488 visualizes a neurovascular bundle. ICG visualizes a lymph node. The neurovascular bundle and the lymph node are identified by visualizing the neurovascular bundle and the lymph node as described above, so that urinary disturbance, defecation disturbance, male function disturbance, or motor function disturbance due to nerve damage in the rectectomy can be reduced. In the case, since two medical agents of ALM-488 and ICG are used, the medical observation system 1 needs a second light source 32 in addition to the first light source 31 as illustrated in FIG. 16. The first light source 31 emits the excitation light L1 that excites one of the two medical agents. The second light source 32 emits the excitation light that excites the other medical agent. Note that the first and second light sources 31 and 32 correspond to the first light source according to the present disclosure. Furthermore, when one medical agent that makes the observation target fluorescent light LF faint among the two medical agents of ALM-488 and ICG is used, the medical observation system 1 positions one of the plurality of regions 13A to 13D of the filter 13 on the optical axis Ax (hereinafter, referred to as first mode). When the other medical agent is used, the medical observation system 1 positions another region of the plurality of regions 13A to 13D on the optical axis Ax (hereinafter, referred to as second mode). Then, a doctor who performs the rectectomy can grasp the position of one of the neurovascular bundle and the lymph node from an image displayed on the display device 7 in the first mode, and grasp the position of the other of the neurovascular bundle and the lymph node from an image displayed on the display device 7 in the second mode.

Moreover, for example, when malignant tumor is excised in malignant tumor excision for treating a childhood brain tumor, ALM-488 and ICG are used as illustrated in Table 1. That is, ALM-488 visualizes a nerve. ICG visualizes a blood vessel (bloodstream). The malignant tumor, the nerve, and the blood vessel are identified by visualizing the nerve and the blood vessel as described above, so that cancer recurrence after the malignant tumor is excised can be prevented, and damage of the nerve and the blood vessel in the malignant tumor excision can be prevented. In the case, since two medical agents of ALM-488 and ICG are used, the medical observation system 1 needs a second light source 32 in addition to the first light source 31 as illustrated in FIG. 16. The first light source 31 emits the excitation light L1 that excites one of the two medical agents. The second light source 32 emits the excitation light L1 that excites the other medical agent. Note that the first and second light sources 31 and 32 correspond to the first light source according to the present disclosure. Furthermore, when one medical agent that makes the observation target fluorescent light LF faint among the two medical agents of ALM-488 and ICG is used, the medical observation system 1 positions one of the plurality of regions 13A to 13D of the filter 13 on the optical axis Ax (hereinafter, referred to as first mode). When the other medical agent is used, the medical observation system 1 positions another region of the plurality of regions 13A to 13D on the optical axis Ax (hereinafter, referred to as second mode). Then, a doctor who performs the malignant tumor excision can grasp the position of one of the nerve and the blood vessel from an image displayed on the display device 7 in the first mode, and grasp the position of the other of the nerve and the blood vessel from an image displayed on the display device 7 in the second mode.

Furthermore, for example, when malignant tumor excision and axillary lymph node dissection are performed in mastectomy for treating breast cancer, ALM-488, LUM-015, and ICG are used as illustrated in Table 1. That is, ALM-488 visualizes a nerve such as an intercostobrachial nerve and a thoracodorsal nerve. LUM-015 visualizes the malignant tumor. ICG visualizes a lymph node. The malignant tumor, the nerve, and the lymph node are identified by visualizing the nerve, the malignant tumor, and the lymph node, so that damage of the nerve in the mastectomy can be reduced. In the case, since three medical agents of ALM-488, LUM-015, and ICG are used, the medical observation system 1 needs the second light source 32 and a third light source 33 in addition to the first light source 31 as illustrated in FIG. 17. The first light source 31 emits the excitation light L1 that excites one of the three medical agents. The second light source 32 emits the excitation light L1 that excites one of the other two medical agents. The third light source 33 emits the excitation light L1 that excites the other of the other two medical agents. Note that the first to third light sources 31 to 33 correspond to the first light source according to the present disclosure. Furthermore, when a medical agent that makes the observation target fluorescent light LF faint among the three medical agents of ALM-488, LUM-015, and ICG is used, the medical observation system 1 positions one of the plurality of regions 13A to 13D of the filter 13 on the optical axis Ax (hereinafter, referred to as first mode). When the other medical agents are used, the medical observation system 1 positions other two regions of the plurality of regions 13A to 13D on the optical axis Ax (hereinafter, referred to as second mode). Then, a doctor who performs the mastectomy can grasp the position of at least one of the nerve, the malignant tumor, and the lymph node from an image displayed on the display device 7 in the first mode, and grasp the positions of the others of the nerve, the malignant tumor, and the lymph node from an image displayed on the display device 7 in the second mode.

Moreover, for example, when a brain tumor is treated by using photo dynamic diagnosis (PDD), ALM-488 and 5-aminolevulinic acid (5-ALA) are used as illustrated in Table 1. That is, fluorescein, which is a fluorescent dye of ALM-488, visualizes the vicinity of a tumor. Protoporphyrin (PpIX) biosynthesized from 5-ALA visualizes tumor cells. Damage in treating a brain tumor can be prevented and the tumor can be safely removed by visualizing tumor cells including the vicinity of a tumor. In the case, since two medical agents of ALM-488 and 5-ALA are used, the medical observation system 1 needs the second light source 32 in addition to the first light source 31 as illustrated in FIG. 16. The first light source 31 emits the excitation light L1 that excites one of the two medical agents. The second light source 32 emits the excitation light L1 that excites the other medical agent. Note that the first and second light sources 31 and 32 correspond to the first light source according to the present disclosure. Furthermore, when 5-ALA that makes the observation target fluorescent light LF faint among the two medical agents of ALM-488 and 5-ALA is used, the medical observation system 1 positions one of the plurality of regions 13A to 13D of the filter 13 on the optical axis Ax (hereinafter, referred to as first mode). When ALM-488 is used, the medical observation system 1 positions another region of the plurality of regions 13A to 13D on the optical axis Ax (hereinafter, referred to as second mode). Then, a doctor who treats a brain tumor can grasp the positions of tumor cells from an image displayed on the display device 7 in the first mode, and grasp the position of the vicinity of the tumor from an image displayed on the display device 7 in the second mode.

Furthermore, for example, when transurethral cystectomy is performed in surgically treating superficial cell cancer on a urinary tract by using the photo dynamic diagnosis, fluorescein and 5-ALA are used as illustrated in Table 1. That is, PpIX biosynthesized from 5-ALA visualizes the tumor. Here, PpIX has a disadvantage of visualizing not only a tumor but highly metabolic inflamed tissues in the bladder. Therefore, fluorescein reduces false positives of the highly metabolic inflamed tissues caused by PpIX. In the case, since two medical agents of fluorescein and 5-ALA are used, the medical observation system 1 needs the second light source 32 in addition to the first light source 31 as illustrated in FIG. 16. The first light source 31 emits the excitation light L1 that excites one of the two medical agents. The second light source 32 emits the excitation light L1 that excites the other medical agent. Note that the first and second light sources 31 and 32 correspond to the first light source according to the present disclosure. Furthermore, when 5-ALA that makes the observation target fluorescent light LF faint among the two medical agents of fluorescein and 5-ALA is used, the medical observation system 1 positions one of the plurality of regions 13A to 13D of the filter 13 on the optical axis Ax (hereinafter, referred to as first mode). When fluorescein is used, the medical observation system 1 positions another region of the plurality of regions 13A to 13D on the optical axis Ax (hereinafter, referred to as second mode). Then, a doctor who performs transurethral cystectomy can grasp the position of a tumor from an image displayed on the display device 7 in the first mode, and grasp a false positive of a highly metabolic inflamed tissue caused by PpIX from an image displayed on the display device 7 in the second mode.

(Variation 2)

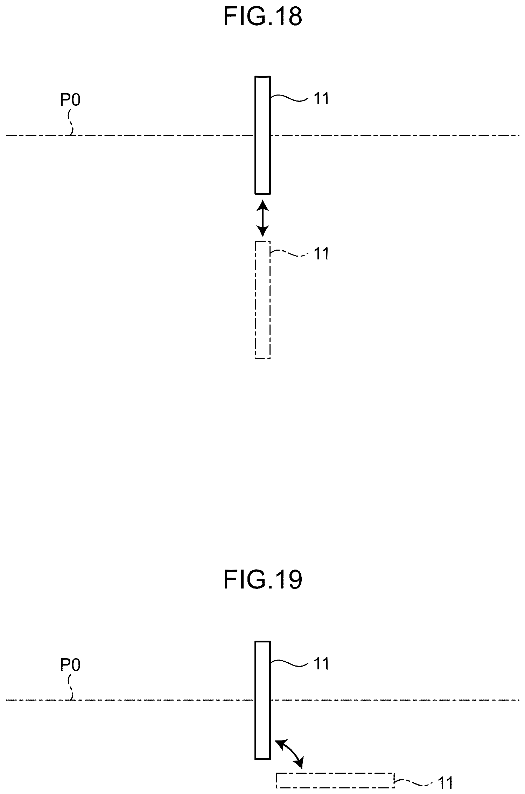

FIG. 18 illustrates Variation 2 of the first and second embodiments. Specifically, FIG. 18 illustrates positions between which the filter 11 according to Variation 2 moves.

Although, in Variation 1 above, the mode (first and second modes) is switched by rotating the filter 13 around the rotation axis RAx, this is not a limitation.

For example, as in Variation 2 in FIG. 18, the filter 11 is positioned at a first position (position of filter 11 indicated by solid line in FIG. 18) on an observation optical path P0 or a second position (position of filter 11 indicated by dash-dot line in FIG. 18) deviated from the observation optical path P0 by operating a movement mechanism (not illustrated). Note that, in Variation 2, the filter 11 moves between the first position and the second position while maintaining the posture in which a light incident surface of the filter 11 intersects with (is, in FIG. 18, orthogonal to) the observation optical path P0. Then, the medical observation system 1 sets the first mode by positioning the filter 11 at the first position. Furthermore, the medical observation system 1 sets the second mode by positioning the filter 11 at the second position.

According to Variation 2 described above, effects similar to those in the above-described first and second embodiments are exhibited.

(Variation 3)

FIG. 19 illustrates Variation 3 of the first and second embodiments. FIG. 19 illustrates positions between which the filter 11 according to Variation 3 moves.

Although, in Variation 1 above, the mode (first and second modes) is switched by rotating the filter 13 around the rotation axis RAx, this is not a limitation.

For example, as in Variation 3 in FIG. 19, the filter 11 is positioned at a first position (position of filter 11 indicated by solid line in FIG. 19) on the observation optical path P0 or a second position (position of filter 11 indicated by dash-dot line in FIG. 19) deviated from the observation optical path P0 by operating the movement mechanism (not illustrated). Note that, in Variation 3, the filter 11 moves between the first position and the second position by rotating with one end side as the rotation center. Then, the medical observation system 1 sets the first mode by positioning the filter 11 at the first position. Furthermore, the medical observation system 1 sets the second mode by positioning the filter 11 at the second position.

According to Variation 3 described above, effects similar to those in the above-described first and second embodiments are exhibited.

(Variation 4)

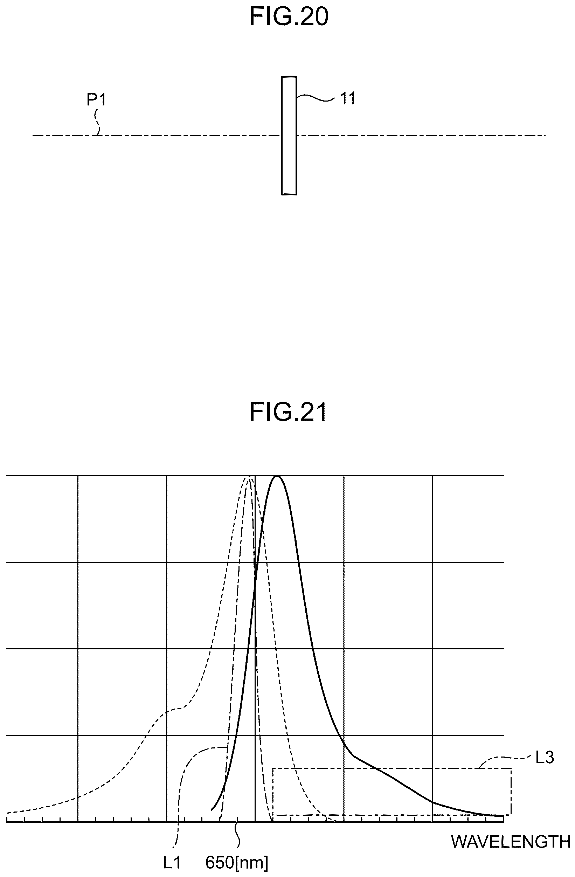

FIGS. 20 and 21 illustrate Variation 4 of the first and second embodiments. Specifically, FIG. 20 illustrates the arrangement position of the filter 11 according to Variation 4. FIG. 21 illustrates the absorption spectrum and the emission spectrum of LUM-015, the spectrum of the excitation light L1, and a wavelength range in which the auto-fluorescent light L3 can be generated at the time when the excitation light L1 propagates through the first optical path P1. Note that, in FIG. 21, the horizontal axis represents a wavelength [nm], and the vertical axis represents absorbance and light intensity such as fluorescence intensity. Furthermore, in FIG. 21, the absorption spectrum of LUM-015 is indicated by a dashed line. The emission spectrum of LUM-015 is indicated by a solid line. The spectrum of the excitation light L1 is indicated by a dash-dot line. The wavelength range in which the auto-fluorescent light L3 can be generated is indicated by a dash-double-dot line.

In Variation 4, a preferred wavelength of the excitation light L1 and preferred characteristics of the filter 11 in a case where the filter 11 is disposed on the first optical path P1 as illustrated in FIG. 20 will be described by using, as an example, a case where LUM-015 is used as a medical agent.

The excitation light L1 preferably has a wavelength in which a large amount of medical agent is absorbed in order to increase the intensity of the observation target fluorescent light LF emitted from the medical agent. Furthermore, the wavelength of the excitation light L1 preferably includes a wavelength range in which the intensity is half or more with respect to the peak wavelength of the absorption spectrum of the medical agent. Moreover, it is efficient that the excitation light L1 has a wavelength of the peak wavelength of the absorption spectrum of the medical agent -10 [nm] to the peak wavelength +10 [nm].

Here, the peak wavelength of the absorption spectrum of LUM-015 is approximately 650 [nm] as illustrated in FIG. 21. Therefore, the excitation light L1 preferably has a wavelength in a range of 640 to 660 [nm].

Furthermore, the auto-fluorescent light L3 is generated when the excitation light L1 propagates through the first optical path P1 on the side of a long wavelength of the wavelength band of the excitation light L1 as illustrated in FIG. 21. Therefore, the filter 11 is preferably designed to perform cutting in a wavelength band of the peak wavelength of the excitation light L1 +10 [nm] to the peak wavelength +310 [nm]. That is, when LUM-015 is used, the filter 11 is preferably designed to perform cutting in a wavelength band of 660 to 960 [nm]. Here, as being closer to the peak wavelength of the excitation light L1, the auto-fluorescent light L3 has higher intensity. Therefore, the OD value of a wavelength band in which cutting is performed in the filter 11 may be changed in accordance with the intensity. For example, the filter 11 is designed such that the OD value of the peak wavelength of the excitation light L1 +10 [nm] to the peak wavelength +150 [nm] is larger than the OD value of the peak wavelength +150 [nm] to the peak wavelength +310 [nm]. In general, setting a constant OD value in a wavelength band in which cutting is performed increases the difficulty level of manufacturing. Therefore, when the OD value is changed in a wavelength band in which cutting is performed as described above, the filter 11 can be easily manufactured, and manufacturing cost can be reduced.

When the filter 11 is disposed on the first optical path P1, a wavelength band in which transmission is performed and a wavelength band in which cutting is performed in the filter 11 are preferably designed as illustrated in Table 2 below not just in a case where LUM-015 is used as a medical agent.

Table 2

| Observation target light | Wavelength band in which transmission is performed | Wavelength band in which cutting is performed |
|---|---|---|
| Observation target fluorescent light | Peak wavelength (hereinafter, referred to as $\lambda 1$) of excitation light -10 [nm] to $\lambda 1$ +10 [nm] | $\lambda 1$ +10 [nm] to $\lambda 1$ +310 [nm] (hereinafter, this wavelength band is referred to as $\lambda 2$) |
| White light | Wavelength band in which identification performance is not deteriorated in clinical use (hereinafter, this wavelength band is referred to as $\lambda 3$) | Long wavelength end of $\lambda 3$ +10 [nm] to long wavelength end +310 [nm] |

(continued)

| Observation target light | Wavelength band in which transmission is performed | Wavelength band in which cutting is performed |
|---|---|---|
| White light and observation target fluorescent light (visible light) | $\lambda3$ (including $\lambda1$) | Long wavelength end of $\lambda3$ +10 [nm] to long wavelength end +310 [nm] |
| White light and observation target fluorescent light (invisible light) | $\lambda1$ +$\lambda3$ | At least $\lambda2$ As necessary, wavelength band between $\lambda1$ -10 [nm] and long wavelength end of $\lambda3$ +10 [nm] |

Specifically, a case where only the observation target fluorescent light LF is set as observation target light is as described above. Furthermore, when only white light for normal light observation is set as observation target light, the filter 11 is preferably designed such that a wavelength band in which identification performance is not deteriorated in clinical use (e.g., wavelength band of approximately 400 to 700 [nm]) is set as a wavelength band in which transmission is performed and a long wavelength end of the wavelength band +10 [nm] to the long wavelength end +310 [nm] is set as a wavelength band in which cutting is performed. Moreover, when white light for normal light observation and the observation target fluorescent light LF which is visible light are set as observation target light, the filter 11 is preferably designed such that the wavelength band in which transmission is performed and the band in which cutting is performed are similar to those in the case where only the white light is set as the observation target light. Furthermore, when white light for normal light observation and the observation target fluorescent light LF which is invisible light are set as observation target light, the filter 11 is preferably designed such that the peak wavelength of excitation light and the wavelength band in which identification performance is not deteriorated in clinical use (e.g., wavelength band of approximately 400 to 700 [nm]) are set as wavelength bands in which transmission is performed and a wavelength band of the peak wavelength of the excitation light +10 [nm] to the peak wavelength +310 [nm] is set as a wavelength band in which cutting is performed. Note that, design may be made, as necessary, such that a wavelength band between the peak wavelength of the excitation light -10 [nm] and the long wavelength end of the wavelength band in which identification performance is not deteriorated in clinical use +10 [nm] is set as a wavelength band in which cutting is performed.

(Variation 5)

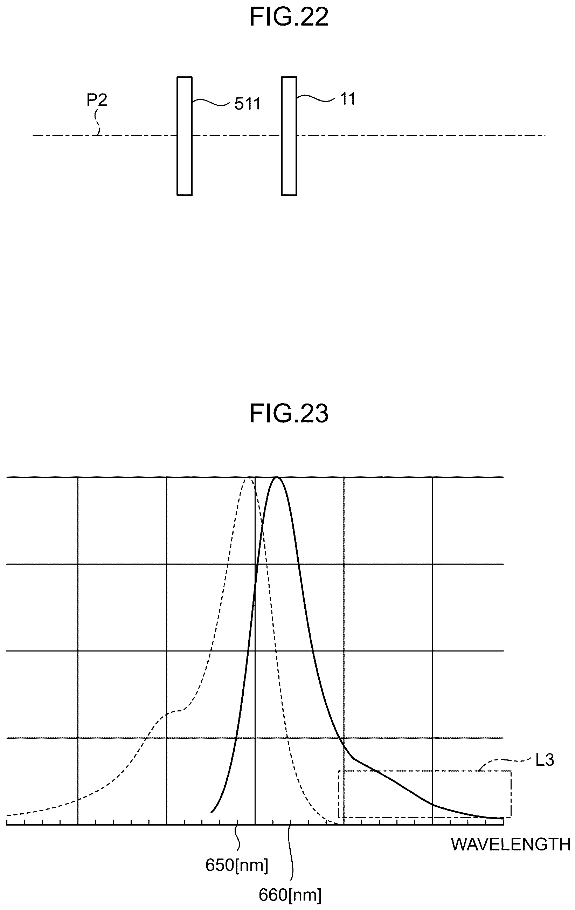

FIGS. 22 and 23 illustrate Variation 5 of the first and second embodiments. Specifically, FIG. 22 illustrates the arrangement position of the filter 11 according to Variation 5. FIG. 23 illustrates the absorption spectrum and the emission spectrum of LUM-015 and a wavelength range in which the auto-fluorescent light L3 can be generated at the time when the second light L2 propagates through the second optical path P2. Note that, in FIG. 23, the horizontal axis represents a wavelength [nm], and the vertical axis represents absorbance and fluorescence intensity. Furthermore, in FIG. 23, the absorption spectrum of LUM-015 is indicated by a dashed line. The emission spectrum of LUM-015 is indicated by a solid line. The wavelength range in which the auto-fluorescent light L3 can be generated is indicated by a dash-double-dot line.

In Variation 5, the preferred characteristics of the filter 11 in a case where the filter 11 is disposed on the second optical path P2 as illustrated in FIG. 22 will be described by using, as an example, the case where LUM-015 is used as a medical agent. Note that the filter 11 is disposed on the side of a further rear stage of the optical path of the excitation light cut filter 511. When the excitation light cut filter 511 is disposed in the insertion portion 2, the filter 11 is only required to be disposed on the side of a further rear stage of the optical path of the excitation light cut filter 511, and can be disposed in, for example, the insertion portion 2, the eyepiece portion 21, or the camera head 5.

The auto-fluorescent light L3 is generated when the second light L2 propagates through the second optical path P2 on the side of a long wavelength of the peak wavelength in the emission spectrum of the medical agent as illustrated in FIG. 23. Therefore, the filter 11 is preferably designed to perform cutting in a wavelength band of the peak wavelength +10 [nm] to the peak wavelength +310 [nm]. Furthermore, when the observation target fluorescent light L3 has an intensity that is not high, design is preferably made such that cutting is performed in a wavelength range in which the intensity is less than half with respect to the peak wavelength of the emission spectrum of the medical agent. Moreover, the OD value is preferably 4 or 5.

Here, as illustrated in FIG. 23, LUM-015 has the peak wavelength of the emission spectrum of approximately 660 [nm]. Therefore, the filter 11 is preferably designed to perform cutting in a wavelength band of 670 to 970 [nm]. Note that the excitation light cut filter 511 preferably has an OD value of 3 or more, and more preferably 4 or 5. Furthermore, the filter 11 preferably has an OD value of 1 or more, and more preferably 2 or 3. Although, in Variation 5, the excitation light cut filter 511 is separated from the filter 11, one filter may have a function of cutting the excitation light L1 and a function of cutting the auto-fluorescent light L3.

When the filter 11 is disposed on the second optical path P2, a wavelength band in which transmission is performed and a wavelength band in which cutting is performed in the filter 11 are preferably designed as illustrated in Table 3 below not just in a case where LUM-015 is used as a medical agent.

Table 3

| Observation target light | Wavelength band in which transmission is performed | Wavelength band in which cutting is performed |
|---|---|---|
| Observation target fluorescent light | Peak wavelength (hereinafter, referred to as $\lambda4$) of emission spectrum of medical agent -10 [nm] to $\lambda4$ +10 [nm] | $\lambda4$ +10 [nm] to $\lambda4$ +310 [nm] (hereinafter, this wavelength band is referred to as $\lambda5$) |
| White light | $\lambda3$ | Long wavelength end of $\lambda3$ +10 [nm] to long wavelength end +310 [nm] |
| White light and observation target fluorescent light (visible light) | $\lambda3$ (including $\lambda4$) | Long wavelength end of $\lambda3$ +10 [nm] to long wavelength end +310 [nm] |
| White light and observation target fluorescent light (invisible light) | $\lambda4 + \lambda3$ | At least $\lambda5$ As necessary, wavelength band between $\lambda4$ -10 [nm] and long wavelength end of $\lambda3$ +10 [nm] |

Specifically, a case where only the observation target fluorescent light LF is set as observation target light is as described above. Furthermore, when only white light for normal light observation is set as observation target light, the filter 11 is preferably designed such that a wavelength band in which identification performance is not deteriorated in clinical use (e.g., wavelength band of approximately 400 to 700 [nm]) is set as a wavelength band in which transmission is performed and a long wavelength end of the wavelength band +10 [nm] to the long wavelength end +310 [nm] is set as a wavelength band in which cutting is performed. Moreover, when white light for normal light observation and the observation target fluorescent light LF which is visible light are set as observation target light, the filter 11 is preferably designed such that the transmission band and the cut band are similar to those in the case where only the white light is set as the observation target light. Furthermore, when white light for normal light observation and the observation target fluorescent light LF which is invisible light are set as observation target light, the filter 11 is preferably designed such that the peak wavelength of the emission spectrum of the medical agent and the wavelength band in which identification performance is not deteriorated in clinical use (e.g., wavelength band of approximately 400 to 700 [nm]) are set as wavelength bands in which transmission is performed and a wavelength band of the peak wavelength of the emission spectrum of the medical agent +10 [nm] to the peak wavelength +310 [nm] is set as a wavelength band in which cutting is performed. Note that, design may be made, as necessary, such that a wavelength band between the peak wavelength of the emission spectrum of the medical agent -10 [nm] and the long wavelength end of the wavelength band in which identification performance is not deteriorated in clinical use +10 [nm] is set as a wavelength band in which cutting is performed.

Note that, in Variations 4 and 5 above, the absorption spectrum and the emission spectrum of the medical agent measured in an experiment using ethanol as a solvent are different from those in blood in a body, for example. Therefore, a wavelength band in which cutting is performed and an OD value in the filter 11 is required to be set in light of a clinical situation.

(Variation 6)

The medical observation system according to Variation 6 uses a so-called video scope (flexible endoscope) including an imaging unit on the distal end side of the insertion portion. For convenience of description, the medical observation system 1 according to Variation 6 will be hereinafter referred to as a medical observation system 1B.

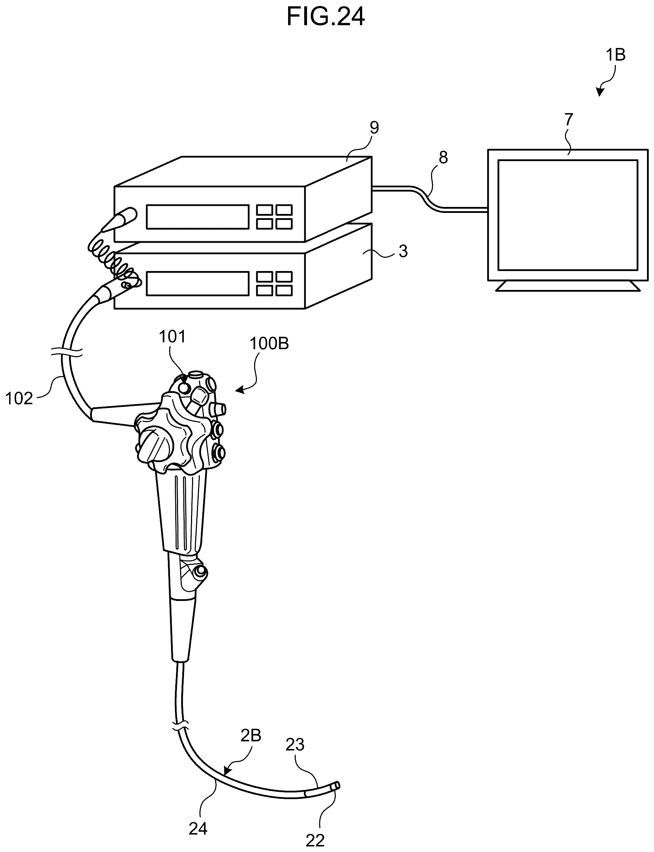

FIG. 24 illustrates Variation 6 of the first and second embodiments.

As illustrated in FIG. 24, the medical observation system 1B includes an endoscope 100B, the light source device 3, the control device 9, and the display device 7. The endoscope 100B captures an in-vivo image of an observed region by inserting an insertion portion 2B into a living body, and outputs the captured image. The light source device 3 emits the first light such as the excitation light L1 from the distal end of the endoscope 100B. The control device 9 processes the captured image output from the endoscope 100B. The display device 7 is connected to the control device 9 via the second transmission cable 8, and displays an image based on a video signal processed by the control device 9.

As illustrated in FIG. 24, the endoscope 100B includes the insertion portion 2B, an operating unit 101, and a universal cord 102. The insertion portion 2B has flexibility and an elongated shape. The operating unit 101 is connected to the proximal end side of the insertion portion 2B, and receives various operations. The universal cord 102 extends from the operating unit 101 in a direction different from the direction in which the insertion portion 2B extends, and incorporates various cables to be connected to the light source device 3 and the control device 9.

As illustrated in FIG. 24, the insertion portion 2B includes a distal end portion 22, a bent portion 23, and a flexible tube portion 24. The bent portion 23 is connected to the proximal end side of the distal end portion 22, includes a plurality of bent pieces, and can be freely bent. The flexible tube portion 24 is connected to the proximal end side of the bent portion 23, and has flexibility and a long shape.

Although not specifically illustrated, the distal end portion 22 incorporates a configuration substantially similar to the camera head 5 described above in the first and second embodiments. Then, the captured image obtained by the distal end portion 22 (imaging unit) is output to the control device 9 via the operating unit 101 and the universal cord 102.

Furthermore, although not specifically illustrated, in the medical observation system 1B, a filter similar to the filters 11 and 12 described above in the first and second embodiments is disposed on the observation optical path including the first optical path and the second optical path. The first light such as the excitation light L1 propagates through the first optical path from the light source device 3 to an observation target. The second light, which is return light of the first light, propagates through the second optical path from the observation target to the imaging element.

Even when the configuration of Variation 6 described above is adopted, effects similar to those of the above-described first and second embodiments are exhibited.

(Variation 7)

The medical observation system according to Variation 7 uses a surgical microscope that enlarges and images a predetermined visual field region inside a subject (living body) or on the surface of the subject (surface of living body) to be observed. For convenience of description, the medical observation system 1 according to Variation 7 will be hereinafter referred to as a medical observation system 1C.

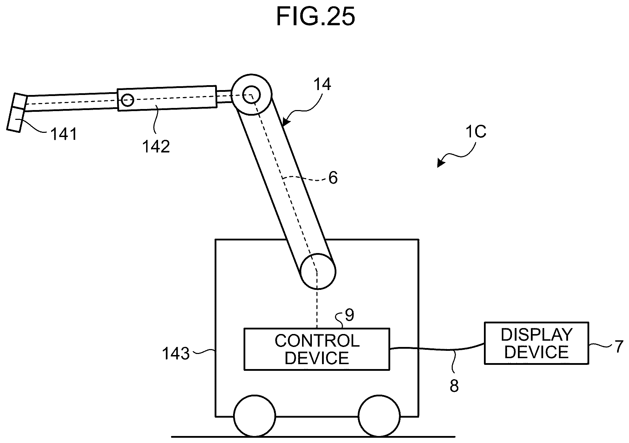

FIG. 25 illustrates Variation 7 of the first and second embodiments.

As illustrated in FIG. 25, the medical observation system 1C includes a surgical microscope 14, the control device 9, and the display device 7. The surgical microscope 14 captures an image for observing a subject, and outputs the captured image. The control device 9 processes the captured image output from the surgical microscope 14. The display device 7 is connected to the control device 9 via the second transmission cable 8, and displays an image based on a video signal processed by the control device 9.

As illustrated in FIG. 25, the surgical microscope 14 includes a microscope portion 141, a support portion 142, and a base portion 143. The microscope portion 141 enlarges and images a minute part of the subject, and outputs the captured image. The support portion 142 is connected to a proximal end portion of the microscope portion 141, and includes an arm that rotatably supports the microscope portion 141. The base portion 143 rotatably holds a proximal end portion of the support portion 142, and can move on a floor surface.

Then, as illustrated in FIG. 25, the control device 9 is installed in the base portion 143. Furthermore, although not specifically illustrated, the light source device 3 is also installed in the base portion 143. The light source device 3 emits the first light such as the excitation light L1 from the surgical microscope 14 to the observation target.

Note that the base portion 143 may be fixed to a ceiling, a wall surface, or the like to support the support portion 142 instead of being movably provided on the floor surface.

Although not specifically illustrated, the microscope portion 141 incorporates a configuration substantially similar to the camera head 5 described above in the first and second embodiments. Then, the captured image obtained by the microscope portion 141 (imaging element) is output to the control device 9 via the first transmission cable 6 arranged along the support portion 142.

Furthermore, although not specifically illustrated, in the medical observation system 1C, a filter similar to the filters 11 and 12 described above in the first and second embodiments is disposed on the observation optical path including the first optical path and the second optical path. The first light such as the excitation light L1 propagates through the first optical path from the light source device 3 to the observation target. The second light, which is return light of the first light, propagates through the second optical path from the observation target to the imaging element.

Even when the configuration of Variation 7 described above is adopted, effects similar to those of the above-described embodiments are exhibited.

(Variation 8)

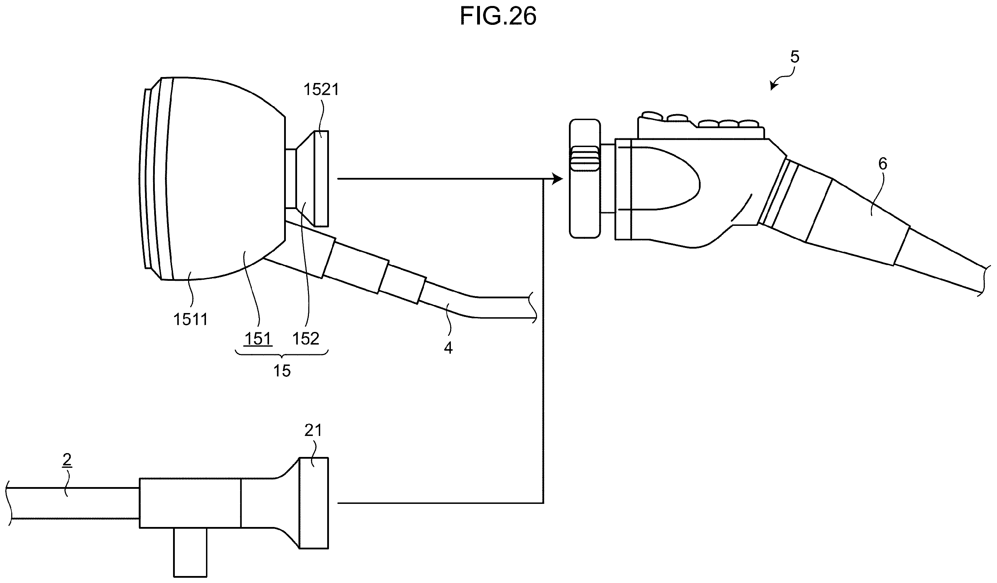

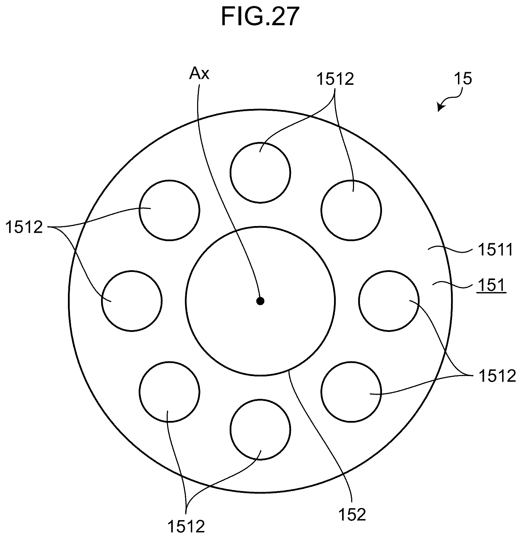

FIGS. 26 and 27 illustrate Variation 8 of the embodiments. Specifically, FIG. 26 illustrates a ring light 15 viewed from a lateral side. Note that the ring light 15 corresponds to an open field observation device for observing a living body tissue. The open field observation device is used in laparotomy. FIG. 27 illustrates the ring light 15 viewed from a front side (left side in FIG. 26).

In Variation 8, in addition to the insertion portion 2 described above in the first and second embodiments, the ring light 15 in FIGS. 26 and 27 is detachably connected to the camera head 5. That is, as illustrated in FIG. 26, the insertion portion 2 or the ring light 15 may be connected to the camera head 5 depending on a state of use of the user.

The ring light 15 is not inserted into the observation target OB like the insertion portion 2. The ring light 15 supplies the first light to a surgical portion, and captures the second light (subject image), which is return light of the first light from the surgical portion. As illustrated in FIGS. 26 and 27, the ring light 15 includes an illumination unit 151 and a subject image capturing unit 152 that captures a subject image.

As illustrated in FIGS. 26 and 27, the illumination unit 151 includes a casing 1511 and a plurality of illumination lenses 1512.

The casing 1511 has an annular shape centered on the optical axis Ax. Then, the other end of the light guide 4 is detachably connected to the casing 1511.

As illustrated in FIG. 27, the plurality of illumination lenses 1512 is arranged at predetermined intervals along the peripheral direction centered on the optical axis Ax on the end surface on the front side of the casing 1511. Then, each of the plurality of illumination lenses 1512 applies the first light to the surgical portion. The first light is supplied from the light source device 3, and introduced into the casing 1511 via the light guide 4.

The subject image capturing unit 152 extends along the optical axis Ax. Furthermore, an optical system is provided in the subject image capturing unit 152. The optical system includes one or a plurality of lenses, and collects the second light (subject image) applied from the plurality of illumination lenses 1512 via the surgical portion. Furthermore, a connection portion 1521 is provided at an end on the proximal end side (right side in FIG. 26) of the subject image capturing unit 152. The connection portion 1521 is designed (has shape) so as to be compatible with the eyepiece portion 21 of the insertion portion 2, and detachably connected to the camera head 5.

Then, in Variation 8, although not specifically illustrated, a filter similar to the filters 11 and 12 described above in the first and second embodiments is disposed on the observation optical path including the first optical path and the second optical path. The first light such as the excitation light L1 propagates through the first optical path from the light source device 3 to the observation target. The second light, which is return light of the first light, propagates through the second optical path from the observation target to the imaging element. More specifically, the filter is provided at a connection portion between the light guide 4 and the ring light 15 or a front surface portion (front surface on side of front stage of optical path) of the subject image capturing unit 152.

Even when the configuration of Variation 8 described above is adopted, effects similar to those of the above-described embodiments are exhibited.

Note that the following configurations also belong to the technical scope of the present disclosure.

(1) A medical observation system including: a light source device configured to emit first light; an imaging element configured to image second light, the second light being return light of the first light from an observation target to which the first light has been applied; and a

   filter disposed on an observation optical path including: a first optical path through which the first light propagates from the light source device to the observation target; and
   a second optical path through which the second light propagates from the observation target to the imaging element, and configured to partially, substantially, or
   completely inhibit unnecessary light containing auto-fluorescent light generated from a member forming the observation optical path by at least one of the first light and the second light.

(2) The medical observation system according to (1) above, in which the filter is disposed on the first optical path, and configured to partially, substantially, or completely inhibit light other than the first light emitted from the light source device.

(3) The medical observation system according to (2) above, further including: an optical visual tube including a part of the first optical path and a part of the second optical path, configured to apply the first light to the observation target and capture the second light; and a light guide including a part of the first optical path and configured to propagate the first light emitted from the light source device to the optical visual tube, in which the filter is disposed at an emission end of the first light in the light source device, an emission end of the first light in the light guide, or an emission end of the first light in the optical visual tube.

(4) The medical observation system according to any one of (1) to (3) above, in which the filter is disposed on the second optical path, and configured to partially, substantially, or completely inhibit light other than observation target light contained in the second light.

(5) The medical observation system according to (4) above, in which the filter is disposed at a position where the second light is substantially parallel light on the second optical path.

(6) The medical observation system according to (4) above, further including: an optical visual tube including a part of the first optical path and a part of the second optical path, configured to apply the first light to the observation target and capture the second light; and a camera head including a part of the second optical path and configured to capture the second light emitted from an eyepiece portion of the optical visual tube, the camera head including the imaging element, in which the filter is disposed at an incident end of the second light in the optical visual tube, an incident end of the second light in the camera head, or an imaging surface of the imaging element.

(7) The medical observation system according to any one of (1) to (6) above, in which the filter is configured to make an S/N ratio of a signal value of observation target light in a pixel signal obtained by imaging of the imaging element more than 1 by partially, substantially, or completely inhibiting unnecessary light containing the auto-fluorescent light.

(8) The medical observation system according to any one of (1) to (7) above, in which the first light contains excitation light that excites a substance contained in the observation target, and the second light contains observation target light, which is fluorescent light emitted from the substance contained in the observation target by applying the excitation light.

(9) The medical observation system according to any one of (1) to (8) above, in which the filter is detachable from a member constituting the observation optical path.

(10) The medical observation system according to any one of (1) to (9) above, further including: an optical visual tube including a part of the first optical path and a part of the second optical path, configured to apply the first light to the observation target and capture the second light; and a camera head including a part of the second optical path and configured to capture the second light emitted from an eyepiece portion of the optical visual tube, the camera head including the imaging element, in which the filter is disposed between the eyepiece portion of the optical visual tube and the camera head on the second optical path.

(11) The medical observation system according to any one of (1) to (10) above, further including: an optical visual tube including a part of the first optical path and a part of the second optical path, configured to apply the first light to the observation target and capture the second light; and a light guide including a part of the first optical path and configured to propagate the first light emitted from the light source device to the optical visual tube, in which the filter is disposed between the optical visual tube and the light guide on the first optical path.

(12) The medical observation system according to any one of (1) to (11) above, further including: an optical visual tube including a part of the first optical path and a part of the second optical path, configured to apply the first light to the observation target and capture the second light; in which the filter is disposed at an emission end that applies the first light in the optical visual tube to the observation target on the first optical path.

(13) The medical observation system according to any one of (1) to (9) above, further including: an optical visual tube including a part of the first optical path and a part of the second optical path, configured to apply the first light to the observation target and capture the second light; a light guide including a part of the first optical path and configured to propagate the first light emitted from the light source device to the optical visual tube; and a camera head including a part of the second optical path and configured to capture the second light emitted from an eyepiece portion of the optical visual tube, the camera head including the imaging element, in which the filter includes: a first filter disposed between the eyepiece portion of the optical visual tube and the camera head on the second optical path; and a second filter disposed between the optical visual tube and the light guide on the first optical path or at an emission end that applies the first light in the optical visual tube to the observation target.

(14) The medical observation system according to any one of (1) to (13) above, in which the light source device includes a first light source configured to emit the first light, and one or a plurality of first light sources is provided.

(15) The medical observation system according to any one of (1) to (14) above, in which the light source device includes a first light source configured to emit the first light, and the first light source includes an LED or a semiconductor laser.

(16) A filter disposed on an observation optical path including: a first optical path through which first light propagates from a light source device configured to emit the first light to an observation target; and a second optical path through which second light propagates, the second light being return light of the first light from the observation target to which the first light has been applied, and configured to partially, substantially, or completely inhibit unnecessary light containing auto-fluorescent light generated from a member forming the observation optical path by at least one of the first light and the second light.

(17) The filter according to (16) above, in which the filter is disposed on the first optical path, and configured to partially, substantially, or completely inhibit light other than the first light emitted from the light source device.

(18) The filter according to (17) above, in which the filter is disposed at an emission end of the first light in the light source device, an emission end of the first light in an optical visual tube including a part of the first optical path and a part of the second optical path, configured to apply the first light to the observation target and capture the second light, or an emission end of the first light in a light guide including a part of the first optical path and configured to propagate the first light emitted from the light source device to the optical visual tube.

(19) The filter according to any one of (16) to (18) above, in which the filter is disposed on the second optical path, and configured to partially, substantially, or completely inhibit light other than observation target light contained in the second light.

(20) The filter according to (19) above, in which the filter is disposed at a position where the second light is substantially parallel light on the second optical path.

(21) The filter according to (19) above, in which the filter is disposed at an incident end of the second light in an optical visual tube including a part of the first optical path and a part of the second optical path, configured to apply the first light to the observation target and capture the second light, an incident end of the second light in a camera head including a part of the second optical path and configured to capture the second light emitted from an eyepiece portion of the optical visual tube, the camera head including an imaging element configured to image the second light, or an imaging surface of the imaging element.

(22) The filter according to any one of (16) to (21) above, in which the filter is configured to make an S/N ratio of a signal value of observation target light in a pixel signal obtained by imaging of an imaging element configured to image the second light more than 1 by partially, substantially, or completely inhibiting unnecessary light containing the auto-fluorescent light.

(23) The filter according to any one of (16) to (22) above, in which the first light contains excitation light that excites a substance contained in the observation target, and the second light contains observation target light, which is fluorescent light emitted from the substance contained in the observation target by applying the excitation light.

(24) The filter according to any one of (16) to (23) above, in which the filter is detachable from a member constituting the observation optical path.

(25) The filter according to any one of (16) to (24) above, in which the filter is disposed, on the second optical path, between an eyepiece portion of an optical visual tube including a part of the first optical path and a part of the second optical path, configured to apply the first light to the observation target and capture the second light and a camera head that includes a part of the second optical path, captures the second light emitted from the eyepiece portion of the optical visual tube, and includes an imaging element configured to image the second light.

(26) The filter according to any one of (16) to (25) above, in which the filter is disposed, on the first optical path, between an optical visual tube including a part of the first optical path and a part of the second optical path, configured to apply the first light to the observation target and capture the second light and a light guide including a part of the first optical path and configured to propagate the first light emitted from the light source device to the optical visual tube.

(27) The filter according to any one of (16) to (26) above, in which the filter is disposed, on the first optical path, at an emission end that applies the first light to the observation target in the optical visual tube including a part of the first optical path and a part of the second optical path, configured to apply the first light to the observation target and capture the second light.

(28) The filter according to any one of (16) to (24) above, in which the filter includes: a first filter disposed, on the second optical path, between an eyepiece portion of an optical visual tube including a part of the first optical path and a part of the second optical path, configured to apply the first light to the observation target and capture the second light and a camera head that includes a part of the second optical path, captures the second light emitted from the eyepiece portion of the optical visual tube, and includes an imaging element configured to image the second light; and a second filter disposed, on the first optical path, between the optical visual tube and a light guide including a part of the first optical path and configured to propagate the first light emitted from the light source device to the optical visual tube or at an emission end that applies the first light in the optical visual tube to the observation target.

(29) An optical visual tube for applying first light to an observation target and capturing second light, the second light being return light of the first light from the observation target to which the first light has been applied, including: a part of a first optical path through which the first light propagates from a light source device configured to emit the first light to the observation target; a part of a second optical path through which the second light propagates; and a filter configured to partially, substantially, or completely inhibit unnecessary light containing auto-fluorescent light generated from a member forming an observation optical path including the first optical path and the second optical path by at least one of the first light and the second light.

(30) An optical visual tube according to (29) above, in which the filter is disposed on the first optical path, and configured to partially, substantially, or completely inhibit light other than the first light emitted from the light source device.

(31) The optical visual tube according to (30) above, in which the filter is disposed at an emission end of the first light in the optical visual tube.

(32) The optical visual tube according to any one of (29) to (31) above, in which the filter is disposed on the second optical path, and configured to partially, substantially, or completely inhibit light other than observation target light contained in the second light.

(33) The optical visual tube according to (32) above, in which the filter is disposed at a position where the second light is substantially parallel light on the second optical path.

(34) The optical visual tube according to (32) above, in which the filter is disposed at an incident end of the second light in the optical visual tube.

(35) The optical visual tube according to any one of (29) to (34) above, in which the filter is configured to make an S/N ratio of a signal value of observation target light in a pixel signal obtained by imaging of an imaging element configured to image the second light more than 1 by partially, substantially, or completely inhibiting unnecessary light containing the auto-fluorescent light.

(36) The optical visual tube according to any one of (29) to (35) above, in which the first light contains excitation light that excites a substance contained in the observation target, and the second light contains observation target light, which is fluorescent light emitted from the substance contained in the observation target by applying the excitation light.

(37) The optical visual tube according to any one of (29) to (36) above, in which the filter is detachable from the optical visual tube.

(38) The optical visual tube according to any one of (29) to (37) above, in which the filter is disposed at an eyepiece portion of the optical visual tube on the second optical path.

(39) The optical visual tube according to any one of (29) to (37), in which the filter includes: a first filter disposed at an eyepiece portion of the optical visual tube on the second optical path; and a second filter disposed at an emission end that applies the first light in the optical visual tube to the observation target on the first optical path.

(40) A light source device configured to emit first light, having a first optical path that propagates the first light and including a filter configured to partially, substantially, or completely inhibit unnecessary light containing auto-fluorescent light generated from a member forming the first optical path by the first light.

(41) The light source device according to (40) above, in which the filter is disposed at an emission end of the first light in the light source device.

(42) The light source device according to (40) or (41) above, in which the filter is configured to make an S/N ratio of a signal value of observation target light in a pixel signal obtained by imaging of an imaging element configured to image second light, the second light being return light of the first light from an observation target to which the first light has been applied, more than 1 by partially, substantially, or completely inhibiting unnecessary light containing the auto-fluorescent light.

(43) The light source device according to any one of (40) to (42) above, in which the first light contains excitation light that excites a substance contained in an observation target, and second light, the second light being return light of the first light from the observation target to which the first light has been applied, contains observation target light, which is fluorescent light emitted from the substance contained in the observation target by applying the excitation light.

(44) The light source device according to any one of (40) to (43) above, in which the filter is detachable from the light source device.

(45) The light source device according to any one of (40) to (44), further including a first light source configured to emit the first light, in which one or a plurality of first light sources is provided.

(46) The light source device according to any one of (40) to (45) above, further including a first light source configured to emit the first light, in which the first light source includes an LED or a semiconductor laser.

(47) A light guide that propagates first light emitted from a light source device, having a part of a first optical path that propagates the first light from the light source device to an observation target and including a filter configured to partially, substantially, or completely inhibit unnecessary light containing auto-fluorescent light generated from a member forming the first optical path by the first light.

(48) The light guide according to (47) above, in which the filter is disposed at an emission end of the first light in the light guide.

(49) The light guide according to (47) or (48) above, in which the filter is configured to make an S/N ratio of a signal value of observation target light in a pixel signal obtained by imaging of an imaging element configured to image second light, the second light being return light of the first light from an observation target to which the first light has been applied, more than 1 by partially, substantially, or completely inhibiting unnecessary light containing the auto-fluorescent light.

(50) The light guide according to any one of (47) to (49) above, in which the first light contains excitation light that excites a substance contained in an observation target, and second light, the second light being return light of the first light from the observation target to which the first light has been applied, contains observation target light, which is fluorescent light emitted from the substance contained in the observation target by applying the excitation light.

(51) The light guide according to any one of (47) to (50) above, in which the filter is detachable from the light guide.

(52) A camera head to which an insertion portion is connected and which includes an imaging element configured to image second light, the insertion portion being to be inserted into an observation target, and capturing the second light, the second light being return light of the first light from the observation target to which first light has been applied, the camera head including a part of a second optical path through which the second light propagates from the observation target to the imaging element, and including a filter configured to partially, substantially, or completely inhibit

EP 4 736 734 A1

unnecessary light containing auto-fluorescent light generated from a member forming an observation optical path including: a first optical path through which the first light propagates from a light source device configured to emit the first light to the observation target; and the second optical path.

(53) The camera head according to (52) above, in which the filter is disposed at a position where the second light is substantially parallel light on the second optical path.

(54) The camera head according to (52) and (53) above, in which the filter is disposed at an incident end of the second light in the camera head.

(55) The camera head according to any one of (52) to (54) above, in which the filter is configured to make an S/N ratio of a signal value of observation target light in a pixel signal obtained by imaging of the imaging element more than 1 by partially, substantially, or completely inhibiting unnecessary light containing the auto-fluorescent light.

(56) The camera head according to any one of (52) to (55) above, in which the first light contains excitation light that excites a substance contained in the observation target, and the second light contains observation target light, which is fluorescent light emitted from the substance contained in the observation target by applying the excitation light.

(57) The camera head according to any one of (52) to (56) above, in which the filter is detachable from the camera head.

(58) A color separation optical device for separating incident light into pieces of light in different wavelength bands, including a filter configured to partially, substantially, or completely inhibit unnecessary light containing auto-fluorescent light generated from a member forming an observation optical path including: a first optical path through which first light propagates from a light source device configured to emit the first light to an observation target; and a second optical path through which second light propagates, the second light being return light of the first light from the observation target to which the first light has been applied.

(59) The color separation optical device according to (58) above, in which the filter is configured to make an S/N ratio of a signal value of observation target light in a pixel signal obtained by imaging of an imaging element configured to image the second light more than 1 by partially, substantially, or completely inhibiting unnecessary light containing the auto-fluorescent light.

(60) The color separation optical device according to (58) or (59) above, in which the first light contains excitation light that excites a substance contained in the observation target, and the second light contains observation target light, which is fluorescent light emitted from the substance contained in the observation target by applying the excitation light.

(61) An imaging element including, on an imaging surface, a filter configured to partially, substantially, or completely inhibit unnecessary light containing auto-fluorescent light generated from a member forming an observation optical path including: a first optical path through which first light propagates from a light source device configured to emit the first light to an observation target; and a second optical path through which second light propagates, the second light being return light of the first light from the observation target to which the first light has been applied.

(62) The imaging element according to (61) above, in which the filter is configured to make an S/N ratio of a signal value of observation target light in a pixel signal obtained by imaging of the imaging element more than 1 by partially, substantially, or completely inhibiting unnecessary light containing the auto-fluorescent light.

(63) The imaging element according to (61) or (62) above, in which the first light contains excitation light that excites a substance contained in the observation target, and the second light contains observation target light, which is fluorescent light emitted from the substance contained in the observation target by applying the excitation light.

Reference Signs List

1, 1A to 1C    MEDICAL OBSERVATION SYSTEM

2, 2B    INSERTION PORTION

3    LIGHT SOURCE DEVICE

4    LIGHT GUIDE

5    CAMERA HEAD

6    FIRST TRANSMISSION CABLE

7    DISPLAY DEVICE

| 8 | SECOND TRANSMISSION CABLE |
|---|---|
| 9 | CONTROL DEVICE |
| 10 | THIRD TRANSMISSION CABLE |
| 11 | FILTER (FIRST FILTER) |
| 12 | SECOND FILTER |
| 13 | FILTER |
| 13A | to 13D REGION |
| 14 | SURGICAL MICROSCOPE |
| 15 | RING LIGHT |
| 21 | EYEPIECE PORTION |
| 22 | DISTAL END PORTION |
| 23 | BENT PORTION |
| 24 | FLEXIBLE TUBE PORTION |
| 31 | FIRST LIGHT SOURCE |
| 41 | EMISSION END |
| 51 | IMAGING UNIT |
| 100B | ENDOSCOPE |
| 101 | OPERATING UNIT |
| 102 | UNIVERSAL CORD |
| 141 | MICROSCOPE PORTION |
| 142 | SUPPORT PORTION |
| 143 | BASE PORTION |
| 151 | ILLUMINATION UNIT |
| 152 | SUBJECT IMAGE CAPTURING UNIT |
| 211 | EMISSION END |
| 511 | EXCITATION LIGHT CUT FILTER |
| 512 | LENS UNIT |
| 513 | IMAGING ELEMENT |
| 1511 | CASING |

| 1512 | ILLUMINATION LENS |
| Ar1 | to Ar3 WAVELENGTH BAND |
| Ax | OPTICAL AXIS |
| L1 | EXCITATION LIGHT |
| L2 | SECOND LIGHT |
| L3 | AUTO-FLUORESCENT LIGHT |
| LF | OBSERVATION TARGET FLUORESCENT LIGHT |
| OB | OBSERVATION TARGET |
| P0 | OBSERVATION OPTICAL PATH |
| P1 | FIRST OPTICAL PATH |
| P2 | SECOND OPTICAL PATH |

**Claims**

1. A medical observation system comprising:

   a light source device configured to emit first light;
   an imaging element configured to image second light, the second light being return light of the first light from an observation target to which the first light has been applied; and
   a filter disposed on an observation optical path including: a first optical path through which the first light propagates from the light source device to the observation target; and a second optical path through which the second light propagates from the observation target to the imaging element, and configured to partially, substantially, or completely inhibit unnecessary light containing auto-fluorescent light generated from a member forming the observation optical path by at least one of the first light and the second light.

2. The medical observation system according to claim 1,
   wherein the filter is disposed on the first optical path, and configured to partially, substantially, or completely inhibit light other than the first light emitted from the light source device.

3. The medical observation system according to claim 2, further comprising:

   an optical visual tube including a part of the first optical path and a part of the second optical path, configured to apply the first light to the observation target and capture the second light; and
   a light guide including a part of the first optical path and configured to propagate the first light emitted from the light source device to the optical visual tube,
   wherein the filter is disposed at an emission end of the first light in the light source device, an emission end of the first light in the light guide, or an emission end of the first light in the optical visual tube.

4. The medical observation system according to claim 1,
   wherein the filter is disposed on the second optical path, and configured to partially, substantially, or completely inhibit light other than observation target light contained in the second light.

5. The medical observation system according to claim 4,
   wherein the filter is disposed at a position where the second light is substantially parallel light on the second optical path.

6. The medical observation system according to claim 4, further comprising:

an optical visual tube including a part of the first optical path and a part of the second optical path, configured to apply the first light to the observation target and capture the second light; and

a camera head including a part of the second optical path and configured to capture the second light emitted from an eyepiece portion of the optical visual tube, the camera head including the imaging element,

wherein the filter is disposed at an incident end of the second light in the optical visual tube, an incident end of the second light in the camera head, or an imaging surface of the imaging element.

7. The medical observation system according to claim 1,

wherein the filter is configured to make an S/N ratio of a signal value of observation target light in a pixel signal obtained by imaging of the imaging element more than 1 by partially, substantially, or completely inhibiting unnecessary light containing the auto-fluorescent light.

8. The medical observation system according to claim 1,

wherein the first light contains excitation light that excites a substance contained in the observation target, and the second light contains observation target light, which is fluorescent light emitted from the substance contained in the observation target by applying the excitation light.

9. The medical observation system according to claim 1,

wherein the filter is detachable from a member constituting the observation optical path.

10. The medical observation system according to claim 1, further comprising:

an optical visual tube including a part of the first optical path and a part of the second optical path, configured to apply the first light to the observation target and capture the second light; and

a camera head including a part of the second optical path and configured to capture the second light emitted from an eyepiece portion of the optical visual tube, the camera head including the imaging element,

wherein the filter is disposed between the eyepiece portion of the optical visual tube and the camera head on the second optical path.

11. The medical observation system according to claim 1, further comprising:

an optical visual tube including a part of the first optical path and a part of the second optical path, configured to apply the first light to the observation target and capture the second light; and

a light guide including a part of the first optical path and configured to propagate the first light emitted from the light source device to the optical visual tube,

wherein the filter is disposed between the optical visual tube and the light guide on the first optical path.

12. The medical observation system according to claim 1, further comprising:

an optical visual tube including a part of the first optical path and a part of the second optical path, configured to apply the first light to the observation target and capture the second light;

wherein the filter is disposed at an emission end that applies the first light in the optical visual tube to the observation target on the first optical path.

13. The medical observation system according to claim 1, further comprising:

an optical visual tube including a part of the first optical path and a part of the second optical path, configured to apply the first light to the observation target and capture the second light;

a light guide including a part of the first optical path and configured to propagate the first light emitted from the light source device to the optical visual tube; and

a camera head including a part of the second optical path and configured to capture the second light emitted from an eyepiece portion of the optical visual tube, the camera head including the imaging element,

wherein the filter includes:

a first filter disposed between the eyepiece portion of the optical visual tube and the camera head on the second optical path; and

a second filter disposed between the optical visual tube and the light guide on the first optical path or at an emission end that applies the first light in the optical visual tube to the observation target.

14. The medical observation system according to claim 1,

    wherein the light source device includes a first light source configured to emit the first light, and one or a plurality of first light sources is provided.

15. The medical observation system according to claim 1,

    wherein the light source device includes a first light source configured to emit the first light, and the first light source includes an LED or a semiconductor laser.

16. A filter disposed on an observation optical path including: a first optical path through which first light propagates from a light source device configured to emit the first light to an observation target; and a second optical path through which second light propagates, the second light being return light of the first light from the observation target to which the first light has been applied, and configured to partially, substantially, or completely inhibit unnecessary light containing auto-fluorescent light generated from a member forming the observation optical path by at least one of the first light and the second light.

17. An optical visual tube for applying first light to an observation target and capturing second light, the second light being return light of the first light from the observation target to which the first light has been applied, comprising:

    a part of a first optical path through which the first light propagates from a light source device configured to emit the first light to the observation target;
    a part of a second optical path through which the second light propagates; and
    a filter configured to partially, substantially, or completely inhibit unnecessary light containing auto-fluorescent light generated from a member forming an observation optical path including the first optical path and the second optical path by at least one of the first light and the second light.

# FIG.1

# FIG.2

# FIG.3

(a)

(b)

# FIG.4

(a)

(b)

# FIG.5

(a)

(b)

## FIG.6

# FIG.7

(a)

OD VALUE

λc    λb    WAVELENGTH

(b)

OD VALUE

λc'    λb'    WAVELENGTH

# FIG.8

# FIG.9

(a)

SIGNAL DENSITY

L1

L3

WAVELENGTH

(b)

SIGNAL DENSITY

L1

L3

WAVELENGTH

(c)

SIGNAL DENSITY

L2

L1

LF

L3

WAVELENGTH

# FIG.10

(a)

(b)

(c)

# FIG.11

(a)

SIGNAL DENSITY

L1

L3

WAVELENGTH

(b)

Ar3

SIGNAL DENSITY

L1

L3

WAVELENGTH

(c)

SIGNAL DENSITY

L1

L2

LF

L3

WAVELENGTH

# FIG.12

(a)

SIGNAL DENSITY

L1

L3

WAVELENGTH

(b)

SIGNAL DENSITY

L1

L3

WAVELENGTH

(c)

SIGNAL DENSITY

L1

L2

LF

L3

WAVELENGTH

# FIG.13

(a)

(b)

(c)

# FIG.14

(a)

SIGNAL DENSITY

L1

L3

WAVELENGTH

(b)

Ar3

L1

SIGNAL DENSITY

L3

WAVELENGTH

(c)

L2

SIGNAL DENSITY

L1

LF

L3

WAVELENGTH

# FIG.15

# FIG.16

# FIG.17

FIG.18

P0

11

11

FIG.19

P0

11

11

# FIG.20

P1
11

# FIG.21

L3

L1   650[nm]

WAVELENGTH

# FIG.22

# FIG.23

# FIG.24

# FIG.25

FIG.26

EP 4 736 734 A1

# FIG.27

## INTERNATIONAL SEARCH REPORT

| | International application No. |
|---|---|
| | **PCT/JP2024/023260** |

### A. CLASSIFICATION OF SUBJECT MATTER

*A61B 1/00*(2006.01)i; *A61B 1/07*(2006.01)i
FI: A61B1/00 511; A61B1/07 731; A61B1/00 731

According to International Patent Classification (IPC) or to both national classification and IPC

### B. FIELDS SEARCHED

Minimum documentation searched (classification system followed by classification symbols)

A61B1/00-1/32

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | JP 2007-29453 A (OLYMPUS MEDICAL SYSTEMS CORP.) 08 February 2007 (2007-02-08) paragraphs [0068]-[0074], fig. 13-19 | 1-8, 10-14, 16-17 |
| Y | | 9, 15 |
| X | JP 2008-43493 A (OLYMPUS CORPORATION) 28 February 2008 (2008-02-28) paragraphs [0014]-[0027], fig. 1 | 1-8, 10-14, 16-17 |
| Y | | 9, 15 |
| Y | WO 2019/176253 A1 (SONY OLYMPUS MEDICAL SOLUTIONS INC.) 19 September 2019 (2019-09-19) paragraphs [0050], [0087], fig. 1, 3D | 9 |
| Y | JP 2019-162231 A (SONY CORPORATION) 26 September 2019 (2019-09-26) paragraph [0174] | 9 |

☑ Further documents are listed in the continuation of Box C.    ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | "&" document member of the same patent family |
| "P" document published prior to the international filing date but later than the priority date claimed | |

| Date of the actual completion of the international search | Date of mailing of the international search report |
|---|---|
| **22 July 2024** | **06 August 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (July 2022)

**INTERNATIONAL SEARCH REPORT**

| International application No. |
| --- |
| **PCT/JP2024/023260** |

### C.    DOCUMENTS CONSIDERED TO BE RELEVANT

| Category* | Citation of document, with indication, where appropriate, of the relevant passages | Relevant to claim No. |
| --- | --- | --- |
| Y | JP 2016-198634 A (OLYMPUS CORPORATION) 01 December 2016 (2016-12-01) paragraph [0052], fig. 2 | 9 |
| Y | JP 2013-46687 A (FUJIFILM CORPORATION) 07 March 2013 (2013-03-07) paragraphs [0032]-[0035] | 15 |
| A | WO 2015/156153 A1 (OLYMPUS CORPORATION) 15 October 2015 (2015-10-15) paragraphs [0068]-[0072] | 1-17 |
| A | JP 2002-10969 A (FUJI PHOTO FILM CO., LTD.) 15 January 2002 (2002-01-15) paragraphs [0005]-[0017] | 1-17 |

Form PCT/ISA/210 (second sheet) (July 2022)

## INTERNATIONAL SEARCH REPORT
### Information on patent family members

International application No.

**PCT/JP2024/023260**

| Patent document cited in search report | | | Publication date (day/month/year) | Patent family member(s) | Publication date (day/month/year) |
|---|---|---|---|---|---|
| JP | 2007-29453 | A | 08 February 2007 | (Family: none) | |
| JP | 2008-43493 | A | 28 February 2008 | (Family: none) | |
| WO | 2019/176253 | A1 | 19 September 2019 | US 2020/0405135 A1 paragraphs [0078], [0122], fig. 1, 3D EP 3753470 A1 paragraphs [0054], [0095], fig. 1, 3D JP 2022-179746 A paragraphs [0050], [0087], fig. 1, 3D | |
| JP | 2019-162231 | A | 26 September 2019 | WO 2019/181149 A1 paragraph [0175] US 2021/0015346 A1 paragraph [0250] | |
| JP | 2016-198634 | A | 01 December 2016 | WO 2015/156153 A1 paragraph [0051], fig. 2 US 2017/0020377 A1 paragraph [0155], fig. 2 EP 3111822 A1 paragraph [0133], fig. 2 | |
| JP | 2013-46687 | A | 07 March 2013 | US 2013/0053703 A1 paragraphs [0057]-[0060] EP 2564760 A1 paragraphs [0020]-[0023] | |
| WO | 2015/156153 | A1 | 15 October 2015 | US 2017/0020377 A1 paragraphs [0182]-[0186] EP 3111822 A1 paragraphs [0160]-[0164] | |
| JP | 2002-10969 | A | 15 January 2002 | US 2003/0045777 A1 paragraphs [0007]-[0023] EP 1149555 A2 paragraphs [0005]-[0018] | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2021132695 A

- JP H6160731 A

**Non-patent literature cited in the description**

- *MEDICAL PHYSICS*, August 2020, vol. 47 (8), 3389-3401